(19) Europäisches Patentamt
European Patent Office
Office européen des brevets

(11) Veröffentlichungsnummer : **0 014 996**
**B1**

(12) # EUROPÄISCHE PATENTSCHRIFT

(45) Veröffentlichungstag der Patentschrift : **28.04.82**

(21) Anmeldenummer : **80100835.0**

(22) Anmeldetag : **20.02.80**

(51) Int. Cl.³ : **C 07 D209/96**, C 07 D401/04, C 07 D401/14, C 07 D413/04, C 07 D417/04, A 61 K 31/40

(54) **Heterocyclische spiroverknüpfte Amidine, ihre Stereoisomeren und optischen Isomeren, Verfahren zu ihrer Herstellung und diese enthaltende Arzneimittel.**

(30) Priorität : **23.02.79 DE 2907070**

(43) Veröffentlichungstag der Anmeldung : **03.09.80 (Patentblatt 80/18)**

(45) Bekanntmachung des Hinweises auf die Patenterteilung : **28.04.82 Patentblatt 82/17**

(84) Benannte Vertragsstaaten : **AT BE CH DE FR GB IT NL SE**

(56) Entgegenhaltungen :
**US - A - 2 931 805**
**US - A - 2 911 409**

(73) Patentinhaber : **HOECHST AKTIENGESELLSCHAFT**
**Postfach 80 03 20**
**D-6230 Frankfurt/Main 80 (DE)**

(72) Erfinder : **Försch, Manfred, Dr.**
**Kranichstrasse 66**
**D-6085 Nauheim (DE)**
Erfinder : **Schaub, Wolfgang, Dr.**
**Luisenstrasse 13**
**D-6233 Kelkheim (Taunus) (DE)**
Erfinder : **Gerhards, Hermann, Dr.**
**Wacholderweg 4**
**D-6238 Hofheim am Taunus (DE)**
Erfinder : **Geyer, Harry Maurice, Dr.**
**Shady Lane 7**
**Road Nr. 8 Broadacres, Flemington, N.J. (US)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

EP 0 014 996 B1

# Heterocyclische spiroverknüpfte Amidine, ihre Stereoisomeren und optischen Isomeren, Verfahren zur ihrer Herstellung und diese enthaltende Arzneimittel

Die Erfindung betrifft eine neue Klasse von heterocyclischen spiroverknüpften Amidinen, die eine starke antidepressive Wirkung besitzen, Verfahren zur Herstellung dieser neuen Verbindungen, sowie pharmazeutische Mittel, welche die erfindungsgemäßen aktiven Verbindungen enthalten.

Gegenstand der Erfindung sind heterocyclische, spiroverknüpfte Amidine der Formel I

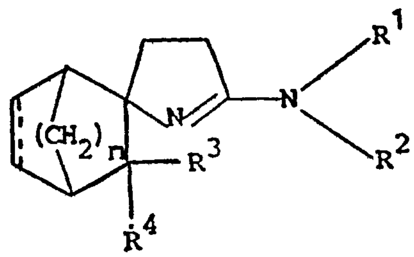

(I)

worin n gleich 1 oder 2 ist ;

$R^1$ und $R^2$ Alkylreste bedeuten, die gemeinsam mit dem Stickstoff einen 5-, 6- oder 7-gliedrigen Ring bilden, worin eines der Kohlenstoffatome durch ein Sauerstoff-, Schwefel- oder Stickstoffatom ersetzt sein kann, wobei das Stickstoffatom substituiert sein kann durch Wasserstoff oder einen Phenylrest, der seinerseits ein- oder mehrfach durch eine $C_1$-$C_4$-Alkyl- oder Alkoxy-, Methylendioxy-, Hydroxy-, Halogen-, Nitro- oder Aminogruppe substituiert sein kann,

$R^3$ gleich Wasserstoff oder Methyl ist und

$R^4$ einen gegebenenfalls durch $C_1$-$C_4$-Alkyl, $C_1$-$C_4$-Alkoxy, Halogen-, oder Trifluormethyl-, ein- oder zweifach substituierten Phenylrest bedeuten ;

sowie die physiologisch verträglichen Salze von Verbindungen der Formel I.

Die erfindungsgemäßen Verbindungen gemäß Formel I können hergestellt werden, indem man

a) eine Verbindung der Formel III

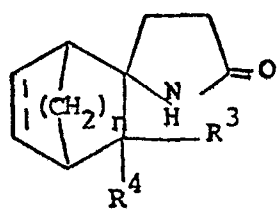

(III)

worin n, $R^3$ und $R^4$ die zur Formel I genannten Bedeutungen haben, mit Phosphoroxichlorid bzw. Phosphorpentoxid und einem Amin der Formel IV,

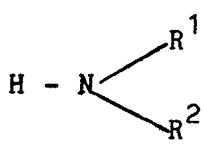

(IV)

worin $R^1$ und $R^2$ die zur Formel I genannte Bedeutung haben, umsetzt ; oder

b) eine Verbindung der Formel II

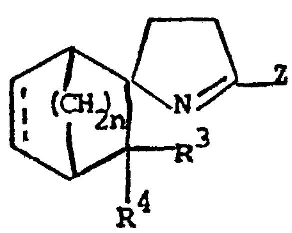

(II)

worin n, $R^3$ und $R^4$ die zur Formel I genannten Bedeutungen haben und Z ein Halogenatom oder einen SH, $SR^5$ oder $OR^5$-Rest bedeutet, wobei $R^5$ einen $C_1$-$C_4$-Alkyl-, einen Benzyl-, Phenylalkyl- oder Phenylsulfonylrest darstellt, mit einem Amin der Formel IV umsetzt ; oder

c) eine Verbindung der Formel V

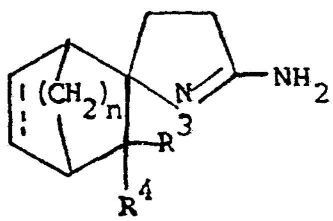

(V)

worin n, $R_3$ und $R_4$ die zur Formel I genannten Bedeutungen haben, mit einem Alkylierungsmittel umsetzt ; oder

d) eine Verbindung der Formel III in trockenem Pyridin bei einer Temperatur von − 30 °C bis Raumtemperatur, vorzugsweise bei − 10 °C mit einem Arylsulfochlorid zu dem intermediären Iminoester umsetzt, der mit der Aminkomponente der Formel IV weiterreagiert.

Die als Zwischenprodukte verwendeten Verbindungen der Formel II werden, wenn

a') Z gleich Halogen ist, hergestellt, indem man eine Verbindung der Formel III mit einem anorganischen Säurehalogenid umsetzt ;

b') Z gleich $SR^5$ ist, hergestellt, indem man eine Verbindung der Formel VI

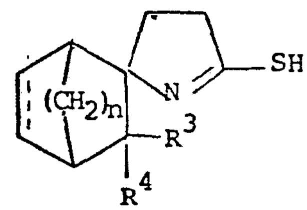

(VI)

worin n, $R_3$ u. $R_4$ die zur Formel I genannten Bedeutungen haben, mit einem Alkylierungsmittel umsetzt ;

c') Z gleich $OR^5$ ist, hergestellt, indem man eine Verbindung der Formel III mit einem Trialkyloxoniumfluoroborat $R_3{}^5OBF_4$ umsetzt ; oder

d') Z gleich $OR^5$ ist, hergestellt, indem man eine Verbindung der Formel III mit einem Säurehalogenid umsetzt und anschließend in situ mit einem Alkalialkoholat behandelt ;

e') Z gleich SH ist, hergestellt, indem man eine Verbindung der Formel III mit Phosphorpentasulfid oder dem Phosphorpentasulfid-Pyridinkomplex zur Reaktion bringt.

Die Herstellung der erfindungsgemäßen Verbindungen gemäß Formel I nach Verfahren a wird bei einer Temperatur von 0-120 °C, vorzugsweise bei 50 °C in einem inerten Lösungsmittel vorzugsweise Benzol, Toluol, Chloroform, Tetrachlorkohlenstoff, Trichlorethylen oder Dioxan durchgeführt.

Bei Verfahren b wird eine Verbindung der Formel II in einem inerten Lösungsmittel, vorzugsweise Diethylenglykol-dimethylether, Dioxan, Dimethylformamid oder Tetrahydrofuran gelöst und entweder in diese Lösung das gasförmige Amin eingeleitet oder zu der Lösung das flüssige bzw. gelöste Amin zugefügt. Man arbeitet dabei, je nach eingesetztem Ausgangsstoff II entweder unter Kühlung, bei Raumtemperatur oder bei mäßig erhöhten Temperaturen.

Verbindungen der Formel II, in denen Z ein Halogenatom, vorzugsweise Cl oder Br bedeutet, können ohne vorherige Isolierung mit dem Amin direkt in der das Imidhalogenid enthaltenden Reaktionslösung umgesetzt werden.

Verbindungen der allgemeinen Formel II, bei denen für Z ein $SR^5$-Rest steht, werden in einem protischen Lösungsmittel vorzugsweise Alkohol, Methanol, Isopropanol mit einem Amin der Formel IV zur Reaktion gebracht. Auch die Verwendung von Dimethylformamid kann vorteilhaft sein. Bei hochsiedenden Aminderivaten der Formel IV kann die Reaktion ohne Lösungsmittel beim Siedepunkt des Amins, vorzugsweise jedoch nicht über 140 °C durchgeführt werden.

Bei Verfahren c wird eine Verbindung der Formel V nach üblichen und bekannten Methoden mit einem Alkylierungsmittel, beispielsweise einem Alkylhalogenid oder einem Alkyl-bzw. Dialkylsulfat oder durch andere übliche Verfahren, beispielsweise nach Leuckart-Wallach umgesetzt.

Die Umsetzung nach Verfahren d wird vorzugsweise in trockenem Pyridin bei einer Temperatur von − 30 °C bis 20 °C, vorzugsweise bei − 10 °C durchgeführt. (H. Henecka, P. Kurtz, Houben-Weyl, Methoden der organischen Chemie, Sauerstoffverbindungen III, Band 8, Seite 704 (1952).

Die als Zwischenprodukte dienende Verbindungen der Formel II werden folgendermaßen gewonnen :

Bei Verfahren a' werden vorzugsweise Phosphorpentahalogenide in einem wasserfreien inerten organischen Lösungsmittel wie Dioxan, Chloroform, Tetrachlorkohlenstoff oder Trichlorethylen bei Temperaturen von − 40 °C bis 50 °C, vorzugsweise bei − 10 °C mit Verbindungen der Formel III zur Reaktion gebracht.

Bei Verfahren b' eignen sich als Alkylierungsmittel Alkylhalogenide, vorzugsweise Methyljodid und Ethylbromid, ferner Diester der Schwefelsäure oder der Toluolsulfonsäure. Als Lösungsmittel werden

vorzugsweise Alkohol, Methanol, Tetrahydrofuran oder Dioxan verwendet, besonders bevorzugt eignet sich Aceton. Die Reaktionstemperatur liegt zwischen 0 °C und dem Siedepunkt des gewählten Lösungsmittels, vorzugsweise bei 30-60 °C.

Das in Verfahren c' verwendete Triethyloxoniumfluoroborat, aus Bortrifluoridetherat und Epichlorhydrin erhältlich (vgl. H. Meerwein et al., J. pr. Chem. (2), *147* 257 (1937) ; *154*, 83 (1939), wird in situ mit einer Verbindung der Formel III zur Reaktion gebracht. Als Lösungsmittel sind insbesondere Diethylether oder Halogenkohlenwasserstoffe z.B. Tetrachlorkohlenstoff geeignet. Die Reaktionstemperatur liegt zwischen 0 °C und dem Siedepunkt des verwendeten Lösungsmittels, vorzugsweise bei 30-40 °C.

Bei Verfahren d' wird das in inertem Lösungsmittel nach Verfahren a' erhältliche Imidhalogenid der Formel II mit Z = Halogen in situ mit einem Alkalialkoholat, vorzugsweise Natriummethylat oder -ethylat umgesetzt. Die Reaktionstemperatur liegt bei 0 °C – 60 °C, vorzugsweise bei 30-40 °C.

Die Umsetzung von Verbindungen der Formel III mit Phosphorpentasulfid nach Verfahren e' wird in einem geeigneten Lösungsmittel wie zum Beispiel Pyridin oder Toluol und Calciumoxid als basischer Katalysator bei 20-120 °C, vorzugsweise bei 50 °C durchgeführt. Es können jedoch auch Verbindungen der Formel III mit der käuflichen Phosphorpentasulfid-Pyridin-Komplexverbindung in einem inerten Lösungsmittel wie Benzol, Chloroform, Methylenchlorid, Tetrahydrofuran oder Dioxan vorzugsweise in Pyridin oder Toluol bei 30-110 °C, vorzugsweise bei 60-80 °C zur Reaktion gebracht werden.

Die Ausgangsstoffe der Formel IX sind, soweit $R^3$ gleich Wasserstoff oder Methyl und $R^4$ gleich Phenyl, Methoxy-phenyl, oder Chlorophenyl sind, in der US-Patentschrift 2 931 805 beschrieben. Verbindungen der Formel IX mit anderen Substituenten für $R^4$ lassen sich in entsprechender Weise nach folgender Reaktion herstellen :

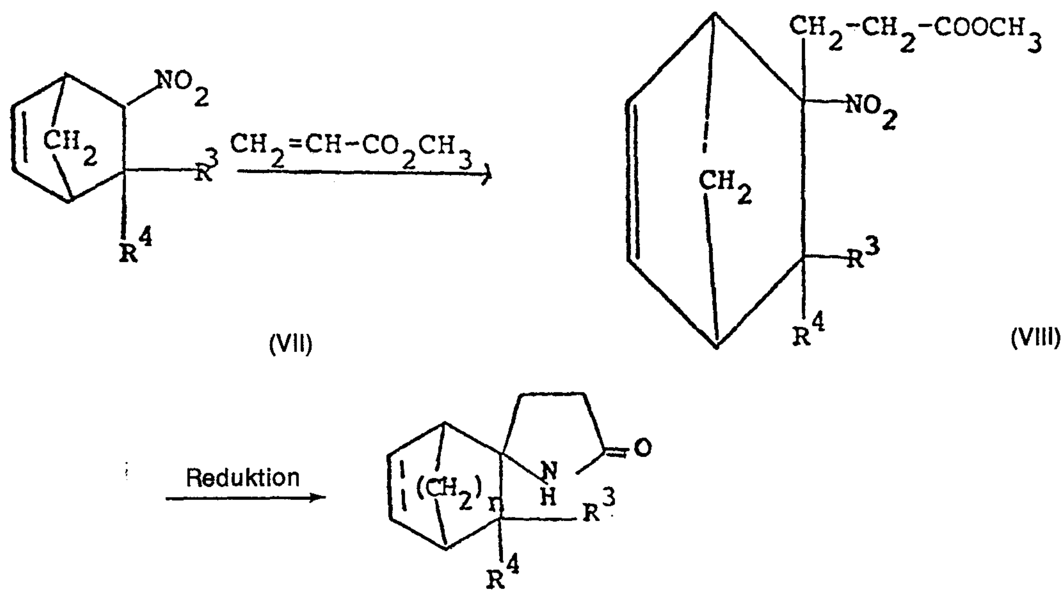


(VII) (VIII) (IX)

Die Nitrobicyclen der Formel VII, worin $R^3$ und $R^4$ die zur Formel I genannte Bedeutung besitzen, lassen sich durch eine Diels-Alder-Reaktion von entsprechenden ω-Nitrostyrolen in siedendem Cyclopentadien darstellen (Lit. J. Org. Chem. *26*, 4898 (1961) ; *8*, 373 (1943) ; J. Amer. Chem. Soc. *73*, 5068 (1951).

Die Michaeladdition von Acrylsäuremethylester an Nitroverbindungen der allgemeinen Formel VII unter Zusatz von Base, vorzugsweise Benzyl-trimethylammoniumhydroxid, wird in Alkohol, Methanol, Tetrahydrofuran vorzugsweise in tert. Butanol oder Dioxan bei 0-120 °C, vorzugsweise bei 10-100 °C durchgeführt.

Ausgangsverbindungen der Formel XI, worin $R^3$, $R^4$ die zur Formel I genannten Bedeutungen haben, lassen sich aus den Bicyclen der Formel X, worin $R^3$ und $R^4$ die zur Formel I genannte Bedeutung besitzen, darstellen :

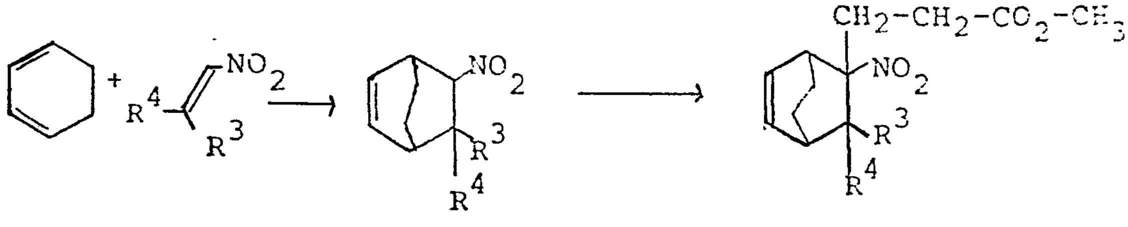

(X)

Reduktion
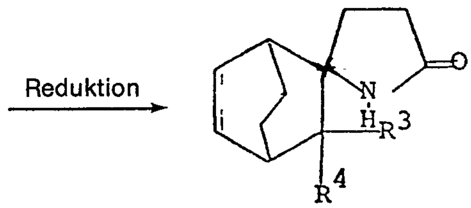
(XI)

Die literaturbekannten 2-Nitrobicyclo [2,2,2] oct-5-ene X sind aus den ω-Nitrostyrolen und einen Überschuß von 1,3-Cyclohexadien darstellbar. Die Ausgangsverbindungen können roh weiterverarbeitet werden.

Als erfindungsgemäße Verbindungen können außer den in den Beispielen genannten vorzugsweise die folgenden hergestellt werden :

3-Phenyl-5′-thiamorpholino-spiro [bicyclo [2,2,1]-heptan-2,2′-5-pyrrolin]
3-(4-Chlorphenyl)-5′-thiamorpholino-spiro [bicyclo [2,2,1]-heptan-2,2′-5-pyrrolin]
3-(4-Bromphenyl)-5′-pyrrolidino-spiro [bicyclo [2,2,1]-heptan-2,2′-5-pyrrolin]
3-(4-Bromphenyl)-5′-piperidino-spiro [bicyclo [2,2,1]-heptan-2,2′-5-pyrrolin]
3-(4-Bromphenyl)-5′-morpholino-spiro [bicyclo [2,2,1]-heptan-2,2′-5-pyrrolin]
5′-Morpholino-3-(4-trifluormethylphenyl)-spiro [bicyclo [2,2,1]-heptan-2,2′-5-pyrrolin]
5′-Piperidino-3-(4-trifluormethylphenyl)-spiro [bicyclo- [2,2,1]-heptan-2,2′-5-pyrrolin]
5′-Pyrrolidino-3-(4-trifluormethylphenyl)-spiro [bicyclo [2,2,1]-heptan-2,2′-5-pyrrolin]
5′-Piperazino-3-(4-trifluormethylphenyl)-spiro [bicyclo [2,2,1]-heptan-2,2′-5-pyrrolin]
3-(4-Methoxyphenyl)-5′-morpholino-spiro [bicyclo [2,2,1]-heptan-2,2′-5-pyrrolin]
3-(4-Methoxyphenyl)-5′-piperidino-spiro [bicyclo [2,2,1]-heptan-2,2′-5-pyrrolin]
3-(4-Ethylphenyl)-5′-morpholino-spiro [bicyclo [2,2,1]-heptan-2,2′-5-pyrrolin]
3-(4-Ethylphenyl)-5′-piperidino-spiro [bicyclo [2,2,1]-heptan-2,2′-5-pyrrolin]
3-(2,3-Dichlorphenyl)-5′-morpholino-spiro [bicyclo [2,2,1]-heptan-2,2′-5-pyrrolin]
3-(2,4-Dichlorphenyl)-5′-pyrrolidino-spiro [bicyclo [2,2,1]-heptan-2,2′-5-pyrrolin]
3-(2,4-Dichlorphenyl)-5′-piperidino-spiro [bicyclo [2,2,1]-heptan-2,2′-5-pyrrolin]
3-(3,4-Dichlorphenyl)-5′-morpholino-spiro [bicyclo [2,2,1]-heptan-2,2′-5-pyrrolin]
3-(3,4-Dichlorphenyl)-5′-piperidino-spiro [bicyclo [2,2,1]-heptan-2,2′-5-pyrrolin]
3-(3,5-Dichlorphenyl)-5′-morpholino-spiro [bicyclo [2,2,1]-heptan-2,2′-5-pyrrolin]
3-Methyl-5′-morpholino-3-phenyl-spiro [bicyclo [2,2,1]-heptan-2,2′-5-pyrrolin]
3-(2-Chlorphenyl)-3-methyl-5′-morpholino-spiro [bicyclo [2,2,1]-heptan-2,2′-5-pyrrolin]
3-(4-Chlorphenyl)-3-methyl-5′-morpholino-spiro [bicyclo [2,2,1]-heptan-2,2′-5-pyrrolin]
3-(4-Chlorphenyl)-3-methyl-5′-piperidino-spiro [bicyclo [2,2,1]-heptan-2,2′-5-pyrrolin]
3-(4-Bromphenyl)-3-methyl-5′-morpholino-spiro [bicyclo [2,2,1]-heptan-2,2′-5-pyrrolin]
3-Methyl-5′-morpholino-3-(4-trifluormethylphenyl)-spiro [bicyclo [2,2,1]-heptan-2,2′-5-pyrrolin]
3-Methyl-5′-piperidino-3-(4-trifluormethylphenyl)-spiro [bicyclo [2,2,1]-heptan-2,2′-5-pyrrolin]
3-(4-Methoxyphenyl)-3-methyl-5′-morpholino-spiro [bicyclo [2,2,1]-heptan-2,2′-5-pyrrolin]
3-(4-Methylphenyl)-3-methyl-5′-morpholino-spiro [bicyclo [2,2,1]-heptan-2,2′-5-pyrrolin]
3-(2,3-Dichlorphenyl)-3-methyl-5′-morpholino-spiro [bicyclo [2,2,1]-heptan-2,2′-5-pyrrolin]
3-(2,4-Dichlorphenyl)-3-methyl-5′-morpholino-spiro [bicyclo [2,2,1]-heptan-2,2′-5-pyrrolin]
3-(3,4-Dichlorphenyl)-3-methyl-5′-morpholino-spiro [bicyclo [2,2,1]-heptan-2,2′-5-pyrrolin]
3-(4-Fluorphenyl)-3-methyl-5′-morpholino-spiro [bicyclo [2,2,1]-heptan-2,2′-5-pyrrolin]
5′-Morpholino-3-phenyl-spiro [bicyclo [2,2,1]-hept-5-en-2,2′-5-pyrrolin]
3-Phenyl-5′-piperidino-spiro [bicyclo [2,2,1]-hept-5-en-2,2′-5-pyrrolin]
3-Phenyl-5′-piperazino-spiro [bicyclo [2,2,1]-hept-5-en-2,2′-5-pyrrolin]
3-(4-Chlorphenyl)-5′-morpholino-spiro [bicyclo [2,2,1]-hept-5-en-2,2′-5-pyrrolin]
3-(4-Chlorphenyl)-5′-piperidino-spiro [bicyclo [2,2,1]-hept-5-en-2,2′-5-pyrrolin]
3-(4-Chlorphenyl)-5′-piperazino-spiro [bicyclo [2,2,1]-hept-5-en-2,2′-5-pyrrolin]
3-(4-Chlorphenyl)-5′-thiamorpholino-spiro [bicyclo [2,2,1]-hept-5-en-2,2′-5-pyrrolin]
3-(4-Methylphenyl)-5′-morpholino-spiro [bicyclo-[2,2,1]-hept-5-en-2,2′-5-pyrrolin]
3-(2,4-Dichlorphenyl)-5′-morpholino-spiro [bicyclo [2,2,1]-hept-5-en-2,2′-5-pyrrolin]
3-(3,4-Dichlorphenyl)-5′-morpholino-spiro [bicyclo [2,2,1]-hept-5-en-2,2′-5-pyrrolin]
3-Methyl-5′-morpholino-3-phenyl-spiro [bicyclo [2,2,1]-hept-5-en-2,2′-5-pyrrolin]
3-(4-Chlorphenyl)-3-methyl-5′-morpholino-spiro [bicyclo [2,2,1]-hept-5-en-2,2′-5-pyrrolin]
3-(4-Chlorphenyl)-3-methyl-5′-piperidino-spiro [bicyclo [2,2,1]-hept-5-en-2,2′-5-pyrrolin]
3-(4-Chlorphenyl)-3-methyl-5′-pyrrolidino-spiro [bicyclo [2,2,1]-hept-5-en-2,2′-5-pyrrolin]
3-(4-Chlorphenyl)-3-methyl-5′-thiamorpholino-spiro [bicyclo [2,2,1]-hept-5-en-2,2′-5-pyrrolin]
3-(4-Chlorphenyl)-5′-morpholino-spiro [bicyclo [2,2,2] octan-2,2′-5-pyrrolin]
3-(4-Chlorphenyl)-5′-piperidino-spiro [bicyclo [2,2,2]-octan-2,2′-5-pyrrolin]
3-(4-Chlorphenyl)-5′-thiamorpholino-spiro [bicyclo [2,2,2] octan-2,2′-5-pyrrolin]
3-(4-Bromphenyl)-5′-morpholino-spiro [bicyclo [2,2,2] octan-2,2′-5-pyrrolin

3-(4-Bromphenyl)-5'-piperidino-spiro [bicyclo [2,2,2] octan-2,2'-5-pyrrolin]
3-(4-Bromphenyl)-5'-thiamorpholino-spiro [bicyclo [2,2,2]-octan-2,2'-5-pyrrolin]
3-(4-Fluorphenyl)-5'-morpholino-spiro [bicyclo [2,2,2]-octan-2,2'-5-pyrrolin]
3-(4-Fluorphenyl)-5'-piperidino-spiro [bicyclo [2,2,2] octan-2,2'-5-pyrrolin]
3-(4-Fluorphenyl)-5'-thiamorpholino-spiro [bicyclo [2,2,2] octan-2,2'-5-pyrrolin]
5'-Morpholino-3-(4-trifluormethylphenyl)-spiro [bicyclo [2,2,2] octan-2,2'-5-pyrrolin]
5'-Piperidino-3-(4-trifluormethylphenyl)-spiro [bicyclo [2,2,2] octan-2,2'-5-pyrrolin]
5'-Thiamorpholino-3-(4-trifluormethylphenyl)-spiro [bicyclo [2,2,2] octan-2,2'-5-pyrrolin]
3-(2,4-Dichlorphenyl)-5'-morpholino-spiro [bicyclo [2,2,2] octan-2,2'-5-pyrrolin]
3-(2,4-Dichlorphenyl)-5'-piperidino-spiro [bicyclo [2,2,2] octan-2,2'-5-pyrrolin]
3-(3,4-Dichlorphenyl)-5'-morpholino-spiro [bicyclo [2,2,2]-octan-2,2'-5-pyrrolin]
3-(3,4-Dichlorphenyl)-5'-piperidino-spiro [bicyclo [2,2,2] octan-2,2'-5-pyrrolin]
3-Methyl-5'-morpholino-3-phenyl-spiro [bicyclo [2,2,2] octan-2,2'-5-pyrrolin]
3-Methyl-3-phenyl-5'-piperidino-spiro [bicyclo [2,2,2] octan-2,2'-5-pyrrolin]
3-Methyl-3-phenyl-5'-thiamorpholino-spiro [bicyclo [2,2,2] octan-2,2'-5-pyrrolin]
3-(4-Chlorphenyl)-3-methyl-5'-morpholino-spiro [bicyclo [2,2,2] octan-2,2'-5-pyrrolin]
3-(4-Chlorphenyl)-3-methyl-5'-piperidino-spiro [bicyclo [2,2,2] octan-2,2'-5-pyrrolin]
3-(4-Chlorphenyl)-3-methyl-5'-thiamorpholino-spiro [bicyclo [2,2,2] octan-2,2'-5-pyrrolin]
5'-Morpholino-3-phenyl-spiro [bicyclo [2,2,2] oct-5-en-2,2'-5-pyrrolin]
3-(4-Chlorphenyl)-5'-morpholino-spiro [bicyclo [2,2,2] oct-5-en-2,2'-5-pyrrolin]
3-(4-Bromphenyl)-5'-morpholino-spiro [bicyclo [2,2,2] oct-5-en-2,2'-5-pyrrolin]
3-(4-Fluorphenyl)-5'-morpholino-spiro [bicyclo [2,2,2]-oct-5-en-2,2'-5-pyrrolin]
5'-Morpholino-3-(4-trifluormethylphenyl)-spiro [bicyclo [2,2,2] oct-5-en-2,2'-5-pyrrolin]
3-(2,4-Dichlorphenyl)-5'-morpholino-spiro [bicyclo [2,2,2] oct-5-en-2,2'-5-pyrrolin]
3-(3,4-Dichlorphenyl)-5'-morpholino-spiro [bicyclo [2,2,2] oct-5-en-2,2'-5-pyrrolin]

Die Verbindungen der Formel I können in vier stereoisomeren Formen vorliegen und jedes Stereoisomere kann in zwei enantiomeren Formen auftreten. Es wurde nun gefunden, daß bei der Herstellung der erfindungsgemäßen Verbindungen der Formel I als Hauptprodukte die stereoisomeren exo-Aryl-endo-Spiropyrrolin-Verbindungen der allgemeinen Formel XII entstehen,

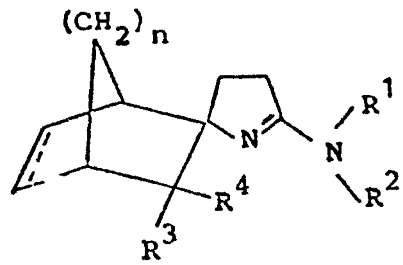

(XII)

worin n, $R^1$, $R^2$, $R^3$ und $R^4$ die zur Formel I genannten Bedeutungen haben. Die Konfiguration der Verbindungen der allgemeinen Formel I wird durch die Stereochemie der als Ausgangsprodukte dienenden Nitrobicyclen der Formel XIII und XIV bestimmt.

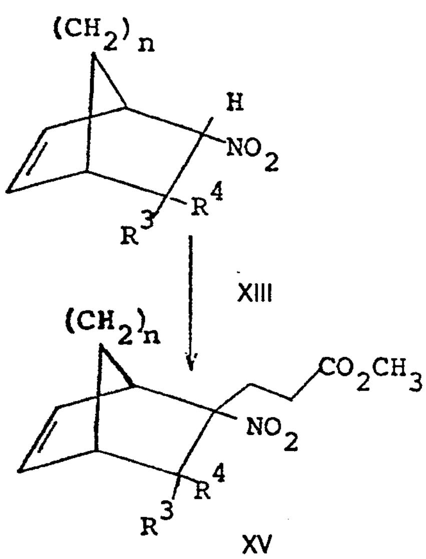

XIII

XV

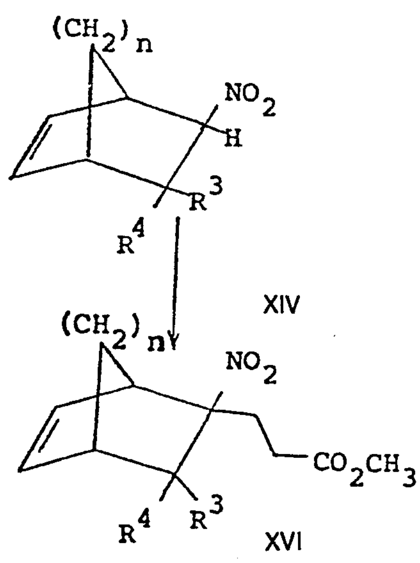

XIV

XVI

Bei der Darstellung dieser Nitrobicyclen fällt in der Regel ein Gemisch der Stereoisomeren XIII und XIV an, wobei das exo-Aryl-endo-nitro-Derivat der Formel XIII als Hauptprodukt vorliegt (1H-NMRspektroskopisch bestimmt). Im Verlauf der weiteren Synthese, wobei das Gemisch der Nitrobicyclen einer Michaeladdition von Acrylester unterworfen wird, fällt der exo-Aryl-endo-nitro-Ester der Formel XV kristallin an, während die endo-Aryl-exo-nitro-Verbindung der Formel XVI in der Mutterlauge zurückbleibt.

Man kann bei der Synthese der erfindungsgemäßen Verbindungen auch von den durch säulenchromatographische Reinigung der Mutterlaugen von exo-Aryl-endo-nitro-Estern der Formel XV erhältlichen isomeren endo-Aryl-exo-nitro-Estern der Formel XVI ausgehen, was bei analogem Syntheseweg zu erfindungsgemäßen Verbindungen führt, deren Stereochemie mit der Formel XVII, als endo-Aryl-exo-pyrrolin-Produkte wiedergegeben werden kann,

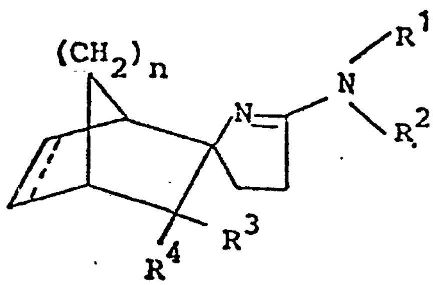

(XVII)

worin n, $R^1$, $R^2$, $R^3$ und $R^4$ die zur Formel I genannten Bedeutungen haben.

Es wurde ferner gefunden, daß die optischen Isomeren der heterocyclischen spiroverknüpften Amidine der Formeln XII und XVII Unterschiede in ihrer biologischen Aktivität aufweisen. So ist beispielsweise die antidepressive Aktivität beim linksdrehenden Isomeren stärker ausgeprägt, während das rechtsdrehende Isomere schwächer wirkt als das Razemat.

Die vorliegende Erfindung betrifft daher auch optisch aktive Stereoisomere von Verbindungen der allgemeinen Formeln XII und XVII, in denen $R^1$, $R^2$, $R^3$ und $R^4$ die in der Formel I angegebenen Bedeutungen besitzen, sowie die Säureadditionssalze davon, Verfahren zur Herstellung dieser Verbindungen und diese enthaltende pharmazeutische Zubereitungen.

Besonders bevorzugte optisch aktive Stereoisomere sind :

(−)-3-exo-(4-Chlorphenyl)-5′-morpholino-spiro [bicyclo [2.2.1]-heptan-2,2′-5-endo-pyrrolin]
(−)-3-endo-(4-Chlorphenyl)-5′-morpholino-spiro [bicyclo [2.2.1]-heptan-2,2′-5-exo-pyrrolin]
(−)-3-exo-(4-Chlorphenyl)-5′-morpholino-spiro [bicyclo [2.2.1]-hept-5-en-2,2′-5-endo-pyrrolin]
(−)-3-endo-(4-Chlorphenyl)-5′-morpholino-spiro [bicyclo [2.2.1]-hept-5-en-2,2′-5-exo-pyrrolin]
(−)-3-exo-(4-Fluorphenyl)-5′-morpholino-spiro [bicyclo [2.2.1]-heptan-2,2′-5-endo-pyrrolin]
(−)-3-endo-(4-Fluorphenyl)-5′-morpholino-spiro [bicyclo [2.2.1]-heptan-2,2′-5-exo-pyrrolin]
(−)-3-exo-(4-Fluorphenyl)-5′-morpholino-spiro [bicyclo [2.2.1]-hept-5-en-2,2′-5-endo-pyrrolin]
(−)-3-endo-(4-Fluorphenyl)-5′-morpholino-spiro [bicyclo-[2.2.1] hept-5-en-2,2′-5-exo-pyrrolin]
3-exo-(4-Bromphenyl)-5′-morpholino-spiro [bicyclo [2.2.1]-heptan-2,2′-5-endo-pyrrolin]
3-endo-(4-Bromphenyl)-5′-morpholino-spiro [bicyclo [2.2.1]-heptan-2.2′-5-exo-pyrrolin]
5′-Morpholino-3-exo-(4-trifluormethylphenyl)-spiro-[bicyclo-[2.2.1] heptan-2,2′-5-endo-pyrrolin]
5′-Morpholino-3-endo-(4-trifluormethylphenyl)-spiro [bicyclo-[2.2.1] heptan-2,2′-5-exo-pyrrolin]

Die erfindungsgemäßen optischen Isomeren können hergestellt werden, indem man entweder eine racemische Verbindung der oben angegebenen Formel XII und XVII, in denen n, $R^1$, $R^2$, $R^3$ und $R^4$ die angegebenen Bedeutungen besitzen, oder eine racemische Vorstufe der oben angegebenen Formeln II, III und V nach einem Standardverfahren in die optischen Isomeren aufspaltet und erforderlichenfalls die so erhaltene optisch aktive Vorstufe zu den gewünschten Verbindungen der Formeln XII oder XVII umwandelt. Vorzugsweise wird eine racemische Mischung einer Verbindung der oben angegebenen Formeln XII und XVII nach einem Standard-Aufspaltungsverfahren, wie es in der Literatur beschrieben ist, in die optischen Isomeren zerlegt.

Selbstverständlich kann die Zerlegung auch mit der racemischen Mischung des Endproduktes oder mit einem Racemat einer Zwischenverbindung der allgemeinen Formel V durchgeführt werden, wobei dann eine oder beide der optischen Isomeren weiter umgesetzt werden.

Im allgemeinen wird die Aufspaltung jedoch bei einer racemischen Mischung der basischen Verbindung der allgemeinen Formel XII oder XVII nach in der Literatur beschriebenen Verfahren, beispielsweise unter Verwendung einer optisch aktiven Säure, durchgeführt. So wird beispielsweise eine Lösung des Racemats in einem geeigneten Losungsmittel, beispielsweise einem Alkohol, mit einer Lösung einer optisch aktiven Säure behandelt, um die Kristallisation des Salzes eines speziellen Enantiomeren zu bewirken Erforderlichenfalls kann das andere Enantiomere oft aus den Mutterlaugen,

gewünschtenfalls durch Behandlung mit einer Base und dann mit dem anderen optischen Isomeren der optisch aktiven Säure hergestellt werden oder eine frische Lösung des Racemats kann auch mit einer Lösung des anderen Enantiomorph der optisch aktiven Säure behandelt werden. Das im Einzelfalle jeweils zu verwendende Lösungsmittel und die jeweils zu verwendende optisch aktive Säure können nicht allgemein vorhergesagt werden und die richtige Wahl wird an Hand von einfachen Versuchen getroffen. Die beste Kombination ist die, mit deren Hilfe es möglich ist, das Salz in hochreinem Zustand (d.h. frei von dem anderen Enantiomeren) und in kristalliner Form zu isolieren.

Es wurde nun gefunden, daß D(−)- und L(+)-Weinsäure, D(−)- und L(+)-Dibenzoylweinsäure sowie D(−) und L(+)-Di-p-tolylweinsäure zur Aufspaltung einiger Verbindungen der Formel XII und XVII besonders gut geeignet sind.

Wenn dann das reine Salz eines Isomeren isoliert worden ist, so wird dieses dann mit einer starken Base, beispielsweise Ammoniumhydroxyd, einer Natriumhydroxyd- oder Natriumcarbonatlösung behandelt, um die freie Base des optisch aktiven Amidines freizusetzen.

Die Verbindungen der Formel I oder ihre Stereoisomeren der Formeln XII oder XVII können nach den beschriebenen Verfahren als solche oder in Form eines Säureadditionssalzes isoliert werden. Bei den Salzen handelt es sich vorzugsweise um die pharmazeutisch verträglichen, nicht-toxischen Additionssalze mit geeigneten Säuren, wie z.B. solchen mit anorganischen Säuren, wie Chlorwasserstoffsäure, Bromwasserstoffsäure und Jodwasserstoffsäure, Salpetersäure, Schwefelsäure oder Phosphorsäure oder mit organischen Säuren, wie Ameisensäure, Essigsäure, Propionsäure, Bernsteinsäure, Weinsäure, Glykolsäure, Milchsäure, Malonsäure, Hydroxymaleinsäure, Fumarsäure, Oxalsäure, Zitronensäure, Apfelsäure, Schleimsäure, Benzoesäure, Salicylsäure, Acetursäure, Embonsäure, Naphthalin-1,5-disulfonsäure, Ascorbinsäure, Phenylessigsäure, p-Amino-salicylsäure, Hydroxyethansulfonsäure, Benzolsulfonsäure oder synthetische Harze, die saure Gruppen enthalten, z.B. solche mit Ionenaustauscherwirkung.

Das dabei erhaltene Säureadditionssalz kann nach bekannten Verfahren in die freie Verbindung umgewandelt werden, beispielsweise durch Behandlung desselben mit einer Base, wie einem Metallhydroxid, Metallalkoholat, Metallcarbonat, mit Ammoniak oder mit einem Hydroxylionenaustauscher oder mit irgendeinem anderen geeigneten Reagens. Ein erhaltenes Säureadditionssalz kann nach bekannten Verfahren in ein anderes Salz umgewandelt werden ; so kann beispielsweise ein Salz mit einer anorganischen Säure mit einem Metallsalz, wie z.B. einem Natrium-, Barium-, oder Silbersalz, einer Säure in einem geeigneten Verdünnungsmittel, in dem das erhaltene anorganische Salz unlöslich ist behandelt und dadurch aus dem Reaktionsmedium entfernt werden. Ein Säureadditionssalz kann auch durch Behandlung mit einem Anionenaustauscherpräparat in ein anderes Säureadditionssalz umgewandelt werden. Ein quartäres Ammoniumsalz kann durch Umsetzung der freien Base mit einem Alkylhalogenid hergestellt werden.

In der US-Patentschrift 2 931 805 sind andere Spironorbornane beschrieben, die eine hypotensive, vasodilatatorische, lokalanaesthetische und ZNS-Wirkung zeigen. Es war aber nicht zu erwarten, daß die erfindungsgemäßen Spiroverbindungen eine starke antidepressive Wirkung haben würden.

Die erfindungsgemäßen Verbindungen und ihre pharmakologisch verträglichen Salze antagonisieren mit einer $ED_{50}$ von 0,1-10 mg/kg intraperitoneal oder oral appliziert die Tetrabenazin-Ptosis an der Maus : Fünf männlichen Tieren mit 22 g-Körpergewicht wird die entsprechende Menge eines 1 %igen Homogenisats der Testsubstanz (in Carboxymethylzellulose) in einer 0,9 %igen NaCl-Lösung (in dest. Wasser) appliziert, wodurch die durch das vorher verabreichte Tetrabenazin bedingte Ptosis aufgehoben wird.

Die antidepressive Wirksamkeit und ihre Brauchbarkeit zur Behandlung von verschiedenen depressiven Zuständen bei Säugetieren wurde weiterhin anhand der Hemmung der Wiederaufnahme von Noradrenalin und Dopamin bei Mäusegehirn-Synaptosomen demonstriert.

Die Verbindungen weisen eine geringe Toxizität auf.

Die $LD_{50}$-Werte liegen im allgemeinen zwischen 100-500 mg/kg per os (Maus).

Die erfindungsgemäßen Verbindungen und ihre pharmazeutisch verträglichen Salze sind innerhalb eines breiten Dosierungsbereiches wirksam, wobei die jeweils verabreichte Dosis von verschiedenen Faktoren, wie z.B. der jeweils verwendeten Verbindung, dem Zustand, dem Typ und der Größe des zu behandelnden Säugetieres, abhängt. Die pro Tag erforderliche Dosis liegt bei der Behandlung von erwachsenen Menschen normalerweise innerhalb des Bereiches von 10-60 mg.

Bei der Behandlung von Testtieren, wie Mäusen und Ratten sind Einzeldosen von 0,1-5 mg pro kg angebracht. Die erfindungsgemäßen aktiven Verbindungen und ihre Salze werden normalerweise oral oder durch Injektion verabreicht und für diesen Zweck werden diese Verbindungen und Salze in der Regel in Form eines pharmazeutischen Präparates verwendet. Diese pharmazeutischen Mittel werden in auf diesem Gebiet an sich bekannter Weise hergestellt und sie enthalten normalerweise mindestens eine erfindungsgemäße Verbindung oder ein Salz davon in Kombination mit einem dafür geeigneten pharmazeutisch verträglichen Träger. Zur Herstellung der erfindungsgemäßen pharmazeutischen Mittel wird der aktive Bestandteil in der Regel mit einem Träger gemischt oder mit einem Träger verdünnt oder von einem Träger eingeschlossen, der in Form einer Kapsel, in Form eines Sachets oder in Form eines anderen Behälters vorliegen kann ; wenn er als Verdünnungsmittel dient, kann es sich dabei um ein festes, halbfestes oder flüssiges Material handeln, das als Verdünnungsmittel, Hilfsstoff oder Medium für

den aktiven Bestandteil (Wirkstoff) dient. Beispiele für geeignete Trager sind Lactose, Dextrose, Saccharose, Sorbit, Mannit, Stärke, Akaziengummi, Calciumphosphat, Alginate, Tragant, Gelatine, Sirup, Methylcellulose, Methyl- und Propylhydroxybenzoat, Talk, Magnesiumstearat oder Mineralöl.

Die erfindungsgemäßen pharmazeutischen Mittel können auf an sich bekannte Weise so formuliert werden, daß sie den Wirkstoff nach der Verabreichung an den Patienten schnell, kontinuierlich oder verzögert abgeben.

Je nach Art der Verabreichung können die oben angegebenen pharmazeutischen Mittel zu Tabletten, Kapseln oder Suspensionen für die orale Verwendung und zu Injektionslösungen für die parenterale Verwendung verarbeitet werden.

Gegenstand der Erfindung ist demgemäß ferner ein pharmazeutisches Mittel (Präparat), das dadurch gekennzeichnet ist, daß es mindestens eine Verbindung der Formel I oder ein pharmazeutisch verträgliches Säureadditionssalz davon in Kombination mit einem dafür geeigneten pharmazeutisch verträglichen Träger enthält.

Beispiel 1

3-(2-Methoxyphenyl)-5'-pyrrolidino-spiro [bicyclo [2,2,1]-heptan-2,2'-5-pyrrolin]-hydrojodid

a) β-[3-(2-Methoxyphenyl)-2-nitro-bicyclo [2,2,1] hept-5-en-2-yl]-propionsäuremethylester

1. 0,1 Mol 3-(2-Methoxyphenyl)-2-nitro-bicyclo [2,2,1]-hept-5-en werden in 600 ml Dioxan unter Zusatz von 12 ml Triton B und 0,1 Mol Acrylsäuremethylester gelöst und bis zum völligen Umsatz zum Sieden erhitzt (DC-Kontrolle). Nach Abziehen der Lösungsmittel wird der Rückstand aus Ethanol unter Zusatz von Tierkohle umkristallisiert. Farblose Kristalle vom Schmp. 72-76 °C.

2. Eine Mischung aus 0,4 Mol 3-(2-Methoxyphenyl)-2-nitro-bicyclo [2,2,1] hept-5-en, 40 ml tert. Butanol und 6 ml Triton B-Lösung (40 %ig in Methanol) werden unter Rühren bei + 5 °C mit 0,4 Mol Acrylsäuremethylester versetzt. Es wird 3 Stunden bei Raumtemperatur nachgerührt, wobei die Temperatur kurzzeitig auf 40 °C steigt. Nach Verdünnen mit Ethanol und Neutralisieren mit wenig verd. Salzsäure wird abgesaugt. Nach Umkristallisation aus Ethanol, farblose Kristalle vom Schmp. 72-76 °C.

b) 3-(2-Methoxyphenyl)-spiro [bicyclo [2,2,1] heptan-2,2'-pyrrolidin]-5'-on

0,2 Mol β-[3-(2-Methoxyphenyl)-2-nitrobicyclo [2,2,1]-hept-5-en-2-yl]-propionsäuremethylester werden in 800 ml Ethanol gelöst und nach Zugabe von 2 Spatel Raney-Nickel 24 h bei 50 °C und 70 bar hydriert. Der Katalysator wird abgesaugt, das Lösungsmittel einrotiert und der Rückstand aus Isopropanol umkristallisiert. Farblose Kristalle vom Schmp. 260 °C.

c) 3-(2-Methoxyphenyl)-spiro [bicyclo [2,2,1] heptan-2,2'-pyrrolidin]-5'-thion

90 mMol 3-(2-Methoxyphenyl)-spiro [bicyclo [2,2,1] heptan-2,2'-pyrrolidin]-5'-on, 10 g Phosphorpentasulfid und 9 g Çalciumoxid werden in 260 ml Toluol aufgeschlämmt und 2 h bei einer Badtemperatur von 50 °C gerührt. Es wird heiß in eine gesättigte Sodalösung filtriert und die Rückstände dreimal mit Toluol ausgekocht. Die unlöslichen Ruckstände werden in konz. Salzsäure gelöst, mit Wasser verdünnt und zusammen mit dem alkalischen Filtrat und den Toluolextrakten in einen Scheidetrichter überführt. Nach kräftigem Schütteln wird die organische Schicht abgetrennt und die salzsaure Phase nochmals mit Toluol extrahiert. Die vereinigten organischen Phasen werden über Natriumsulfat getrocknet und im Vakuum eingeengt. Umkristallisation aus Essigester liefert farblose Kristalle vom Schmp. 194 °C.

d) 3-(2-Methoxyphenyl)-5'-methylthio-spiro [bicyclo [2,2,1]-heptan-2,2'-5-pyrrolin]-hydrojodid

56 mMol 3-(2-Methoxyphenyl)-spiro [bicyclo [2,2,1] hepta-2,2'-pyrrolidin]-5'-thion und 7 ml Methyljodid werden in 220 ml Aceton 10 min. refluxiert. Nach Erkalten wird der Rückstand abgesaugt. Schwachgelbe Verbindung vom Schmp. 228 °C (Zers.).

e) 3-(2-Methoxyphenyl)-5'-pyrrolidino-spiro [bicyclo [2,2,1] heptan-2,2'-5-pyrrolin]-hydrojodid

12 mMol 3-(2-Methoxyphenyl)-5'-methylthio-spiro [bicyclo [2,2,1]-heptan-2,2'-5-pyrrolin]-hydrojodid und 2 g Pyrrolidin werden in 50 ml Ethanol refluxiert. Das Lösungsmittel wird abgezogen und der anfallende Rückstand aus Isopropanol umkristallisiert. Farblose Kristalle vom Schmp. 228 °C.

Die so hergestellte Verbindung ist stereochemisch eine exo-Aryl-endo-Spiropyrrolin Verbindung wie auch alle anderen Verbindungen der Tabelle 1, die analog hergestellt wurden.

Wenn nichts anderes angegeben, besitzen im folgenden die heterocyclischen spiroverknüpften Amidine und ihre Vorstufen exo-Aryl-endo-pyrrolin-Konfiguration.

Tabelle I

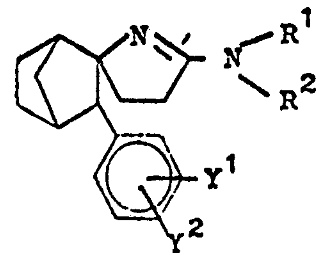

| Beispiel | $Y^1$ | $Y^2$ | 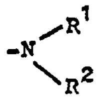 | Salz | Schmp./Zers. |
|---|---|---|---|---|---|
| 2 | H | H | 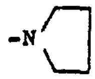 | hydrogenoxalat | 96°C |
| 3 | H | H | 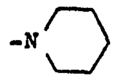 | DL-hydrogentartrat | 238 °C |
| 4 | H | H | 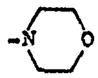 | hydrogen-maleinat | 173°C |
| 5 | H | H | 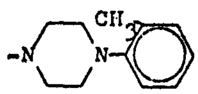 | HJ | 245°C |
| 6 | H | 2 Cl- | 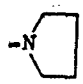 | hydrogenfumarat | 230°C |
| 7 | H | 2 Cl- | 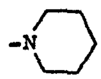 | hydrogennaph-thalin-1,5-di-sulfonat | 278 °C |
| 8 | H | 2 Cl | 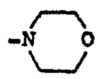 | DL-hydrogentartrat | 225°C |
| 9 | H | 2-$OCH_3$ | 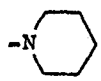 | hydrogennaph-thalin-1,5-di-sulfonat | 255°C |
| 10 | H | 2-$OCH_3$ | 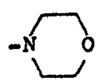 | - | 127°C |
| 11 | 3-$OCH_3$ | 4-$OCH_3$ | 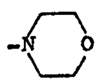 | - | 114-116°C |
| 12 | H | 4-Cl | 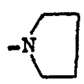 | D,L- hydrogentartrat | 236°C |
| 13 | H | 4-Cl | 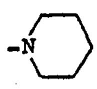 | D,L- hydrogentartrat | 218-220°C |
| 14 | H | 4-Cl | 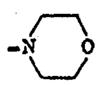 | D,L- hydrogentartrat | 237° (Zers.) |
| 15 | H | 4-Cl | 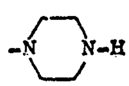 | D,L- hydrogentartrat | 205°C |
| 16 | H | 4-Cl | 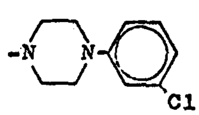 | DL-hydrogentartrat | 225-232°C |
| 17 | H | 4-Cl | 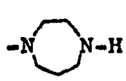 | DL-dihydrogen-tartrat | 223°C |

(Fortsetzung)

| Beispiel | $Y^1$ | $Y^2$ | 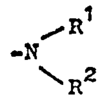 | Salz | Schmp./Zers. |
|---|---|---|---|---|---|
| 18 | H | $4-CH_3$ | 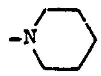 | DL-hydrogentartrat | 189-191°C |
| 19 | H | $4-CH_3$ | 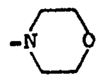 | DL- hydrogentartrat | 235-236°C |
| 20 | H | 4-Cl | 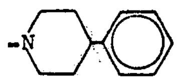 | HJ | 223-224°C |
| 21 | H | 4-F | 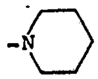 | DL- hydrogentartrat | 220-221°C |
| 22 | H | 4-F | 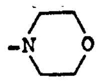 | DL- hydrogentartrat | 234°C |
| 23 | 3-Cl | 4-Cl | 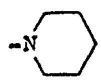 | HJ | 224-226° |
| 24 | 3-Cl | 4-Cl | 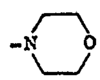 | HJ | 224-225° |
| 25 | 2-Cl | 4-Cl | 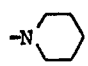 | DL-hydrogentartrat | 199-201°C |
| 26 | 2-Cl | 4-Cl | 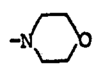 | DL-hydrogentartrat | 202-205°C |
| 27 | 4-Br | H | 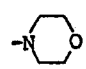 | D,L-hydrogentartrat | 225-227°C |
| 28 | 4-F | H | 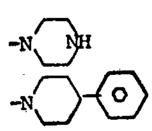 | D,L-hydrogentartrat | 212°C Zers. |
| 29 | 4-F | H | | hydrojodid | 220-224°C |
| 30 | 3-Cl | H | 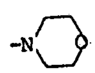 | | 170°C |
| 31 | 4-Cl | H | 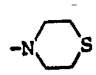 | D,L-hydrogentartrat | 233°C Zers. |
| 32 | 4-Cl | H | -homopiperidino | D,L-hydrogentartrat | 248°C Zers. |
| 33 | $4-OCH_3$ | H | 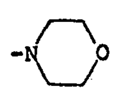 | HJ | 268-270°C |
| 34 | $4-OCH_3$ | H | 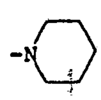 | hydrogentartrat (D,L) | 203-204°C |
| 35 | $4-OC_2H_5$ | H | 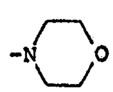 | hydrogentartrat (D,L) | 248°C Zers. |

(Fortsetzung)

| Beispiel | $Y^1$ | $Y^2$ | 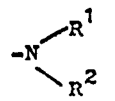 | Salz | Schmp./Zers. |
|---|---|---|---|---|---|
| 36 | $4\text{-}OC_2H_5$ | H | 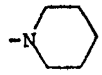 | hydrogentartrat (D,L) | 206 Zers. |
| 37 | $4\text{-}CF_3$ | H | 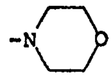 | HJ | 254-255°C |
| 38 | $4\text{-}CF_3$ | H | 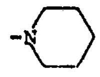 | hydrogentartrat (D,L) | 220°C Zers. |

Analog zu Beispiel 1 wird auch die Verbindungen von Beispiel 39 hergestellt :

Beispiel 39

5'-Morpholino-3-exo-thienyl-spiro [bicyclo [2.2.1]-heptan-2,2'-5-endo-pyrrolin]-hydrojodid, schwach gelbe Kristalle vom Schmp. 137-140 °C.

Beispiel 40

5'-Morpholino-3-phenyl-spiro [bicyclo [2,2,1] hept-5-en-2,2'-5-pyrrolin]-heminaphthalin-1,5 disulfonat

a) 3-Phenyl-spiro [bicyclo [2,2,1] hept-5-en-2,2'-pyrrolidin]-5'-on

0,17 Mol 2-Nitro-3-phenyl [bicyclo [2,2,1] hept-5-en-2-yl]-propionsäuremethylester (nach Lit. US Pat. 2 931 805) und 100 g Zinnpulver werden in 1,3 l Eisessig 3 h bei 100-110 °C gerührt. Nach Abkühlen wird mit Sodalösung neutralisiert und mit Ether zweimal extrahiert. Die organische Phase wird abgetrennt, über Natriumsulfat getrocknet und im Vakuum eingedampft. Umkristallisation aus wenig Methanol liefert farblose Kristalle vom Schmp. 152-155 °C.

b) 3-Phenyl-spiro [bicyclo [2,2,1] hept-5-en-2,2'-pyrrolidin]-5'-thion

20 mMol 3-Phenyl-spiro [bicyclo [2,2,1]-hept-5-en-2,2'-pyrrolidin]-5'-on, 4 g Phosphorpentasulfid, 3 g Calciumoxid werden 2 h bei 50 °C gerührt und heiß in eine ges. Sodalösung filtriert. Der Rückstand wird mit konz. Salzsäure weitgehend zersetzt, mit Wasser verdünnt und die Lösungen vereint. Die Mischung wird mehrmals mit Toluol ausgeschüttelt, die organ. Phasen abgetrennt, getrocknet und im Vakuum eingedampft. Umkristallisation des Rückstandes aus Essigester liefert farblose Kristalle vom Schmp. 204 °C (Zers.).

c) 5'-Methylthio-3-phenyl-spiro [bicyclo [2,2,1] hept-5-en-2,2'-5-pyrrolin]-hydrojodid

6 mMol 3-Phenyl-spiro [bicyclo [2,2,1] hept-5-en-2,2'-pyrrolidin]-5'-thion werden in 30 ml Aceton gelöst, mit 1 ml Methyljodid versetzt und 10 Min. refluxiert. Nach Abkühlen wird der Niederschlag abfiltriert. Die DC-reine kristalline Verbindung wird sofort weiterverarbeitet.

d) 5'-Morpholino-3-phenyl-spiro [bicyclo [2,2,1] hept-5-en-2,2'-5-pyrrolin]-heminaphthalin-1,5-disulfonat

5 mMol 5'-Methylthio-3-phenyl-spiro [bicyclo [2,2,1] hept-5-en-2,2'-5-pyrrolin]-hydrojodid und 2 ml Morpholin in 30 ml Ethanol werden 1 h refluxiert und das Lösungsmittel abgezogen. Nach Ausschütteln mit gesätt. Sodalösung/Ether wird die organ. Phase abgetrennt, getrocknet und einrotiert. Rückstand liefert nach Überführung in das Additionssalz der 1,5-Naphthalindisulfonsäure farblose Kristalle vom Zersetzungspunkt 261 °C (aus Methanol).

Beispiel 41

Analog zu Beispiel 40 läßt sich 3-Phenyl-5'-piperidinospiro [bicyclo [2,2,1] hept-5-en-2,2'-5-pyrrolin]-heminaphthalin-1,5-disulfonat, farblose Kristalle vom Schmp. 176 °C darstellen.

Analog zu Beispiel 40 wurden die Verbindungen der folgenden Beispiele 42 und 43 hergestellt.

Beispiel 42

3-exo-(4-Chlorphenyl)-5'-morpholino-spiro [bicyclo [2,2,2]-hept-5-en-2,2'-5-endo-pyrrolin]-hydrojodid, farblose Kristalle vom Schmp. 253 °C Zers.

Beispiel 43

3-exo-(4-Chlorphenyl)-5'-piperidino-spiro [bicyclo [2,2,1]-hept-5-en-2,2'-5-endo-pyrrolin]-hydrojodid, farblose Kristalle vom Schmp. 190 °C.

Beispiel 44

5'-Morpholino-3-phenyl-spiro [bicyclo [2,2,2] octan-2,2'-5-pyrrolin]-heminaphthalin-1,5-disulfonat

a) 2-Nitro-3-phenyl-bicyclo [2,2,2] oct-5-en

45,1 g 1,3-Cyclohexadien (EMKA), 41,9 g ω-Nitrostyrol, 60 g O-Xylol und 1 Spatelspitze Hydrochinon werden 24 h bei 200 °C und 60 bar Stickstoffdruck im Autoklaven behandelt. Das Lösungsmittel wird abgezogen und der Rückstand in Hochvakuum destilliert. $Sdp_{0,05}$ = 120-160 °C.

b) β-(2-Nitro-3-phenyl-bicyclo [2,2,2] oct-5-en-2-yl)-propionsäuremethylester

Variante A : 0,14 Mol 2-Nitro-3-phenyl-bicyclo [2,2,2]-oct-5-en werden in 50 ml Dioxan unter Zusatz von 27 ml Triton B und 0,14 Mol Acrylsäuremethylester gelöst und bis zum völligen Umsatz refluxiert (4 Stdn). Nach Abziehen der Lösungsmittel kristallisiert der Rückstand durch. Umkristallisation aus Isopropanol liefert Kristalle vom Schmp. 127-132 °C.

Variante B : 0,18 Mol 2-Nitro-3-phenyl-bicyclo [2,2,2] oct-5-en, 0,18 Mol Acrylsäuremethylester und 3,8 ml Triton B in 50 ml tert. Butanol werden 24 h gerührt, dann nochmals mit 0,18 Mol Acrylsäuremethylester und 70 ml Dioxan versetzt und 24 h zum Rückfluß erhitzt. Nach Abkühlen kristallisiert das Reaktionsgemisch aus. Zugabe von wenig Ethanol, Neutralisation mit 2 N Salzsäure und Absaugen des Kristallbreis. Umkristallisation aus Isopropanol unter Zusatz von Tierkohle liefert Kristalle vom Schmp. 127-131 °C.

c) 3-Phenyl-spiro [bicyclo [2,2,2] octan-2,2'-pyrrolidin]-5'-on

55 mMol β-(2-Nitro-3-phenyl-bicyclo [2,2,2] oct-5-en-2yl)-propionsäuremethylester werden in 100 ml Ethanol mit 3 g Raney-Nickel bei 50 °C und 70 bar hydriert. Der Katalysator wird abgesaugt, das Lösungsmittel eingedampft und der Rückstand aus Isopranol umkristallisiert.

d) 3-Phenyl-spiro [bicyclo [2,2,2] octan-2,2'-pyrrolidin]-5'-thion

33 mMol 3-Phenyl-spiro [bicyclo [2,2,2] octan-2,2'-pyrrolidin]-5'-on werden in 200 ml Toluol verteilt und ein Gemisch aus 7,3 g Phosphorpentasulfid und 7 g Calciumoxid unter Rühren zugegeben und 2 1/2 h bei 50 °C gerührt. Die heiße Reaktionslösung wird in eine gesättigte Sodalösung filtriert. Die Rückstände werden in konz. Salzsäure gelöst, mit Wasser verdünnt und mit dem Toluol-Soda-Gemisch vorsichtig vereint. Die wässrige Phase wird mehrmals mit Toluol extrahiert, die Extrakte vereint, getrocknet und im Vakuum eingeengt. Umkristallisation des Rückstandes aus wenig Essigester liefert farblose Kristalle.

e) 5'-Methylthio-3-phenyl-spiro [bicyclo [2,2,2] octan-2,2'-5-pyrrolin]-hydrojodid

24 mMol 3-Phenyl-spiro [bicyclo [2,2,2] octan-2,2'-pyrrolidin]-5'-thion und 3 ml Methyljodid werden 10 Min. in 50 ml Aceton erhitzt. Nach Abkühlen wird abgesaugt. Kristalle vom Schmp. 192-193 °C.

f) 5'-morpholino-3-phenyl-spiro [bicyclo [2,2,2] octan-2,2'-5-pyrrolin]-heminaphthalin-1,5-disulfonat

10 mMol 5'-Methylthio-3-phenyl-spiro [bicyclo [2,2,2] octan-2,2'-5-pyrrolin]-hydrojodid und 2 g Morpholin in 80 ml Ethanol werden 9 h refluxiert, das Lösungsmittel abgezogen und der Rückstand mit einem Gemisch aus 4 N Kalilauge und Essigester behandelt. Die organ. Phase wird abgetrennt, getrocknet und einrotiert. Das Additionssalz der Naphthalin-1,5-disulfonsäure schmilzt bei 299-300 °C.

Analog zu Beispiel 44 werden die Verbindungen der Beispiele 45 und 46 hergestellt.

Beispiel 45

3-exo-(4-Chlorphenyl)-5'-morpholino-spiro [bicyclo [2,2,2]-octan-2,2'-5-endo-pyrrolin] schwach gelbes Öl mit $R_f$ = 0,23, auf Kieselgel-DC, $CHCl_3$/EtOH = 9 : 1.

Beispiel 46

5'-Piperidino-3-phenyl-spiro [bicyclo [2,2,2] octan-2,2'-5-pyrrolin]-heminaphthalin-1,5-disulfonathemihydrat vom Schmp. 298-299 °C.

Beispiel 47

3-endo-(4-Chlorphenyl)-5'-morpholino-spiro [bicyclo [2,2,1]-heptan-2,2'-5-exo-pyrrolin]-hydrojodid

Durch säulenchromatographische Reinigung der ethanolischen Mutterlauge von β-[-exo-(4-Chlorphenyl)-2-endo-nitrobicyclo [2,2,1] hept-5-en-2-yl]-propionsäure-methylester (nach Beispiel 32 und Beispiel 1a) läßt sich der stereoisomere β-[3-endo-(4-Chlorphenyl)-2-exo-nitrobicyclo-[2,2,1]-hept-5-en-2-yl]-propionsäuremethylester als farbloses Öl anreichern (Kieselgel, Eluationsmittel Methylenchlorid).

Völlig analog zur 3-exo-(4-Chlorphenyl)-5'-morpholinospiro [bicyclo [2,2,1] heptan-2,2'-5-endo-pyrrolin]-Verbindung läßt sich daraus das entsprechende stereoisomere endo-Aryl-exo-pyrrolin-Amidinehydrojodid darstellen, farblose Kristalle vom Schmp. 279 °C Zers., aus Ethanol umkristallisiert.

Beispiel 48

(−)-3-exo-(4-Chlorphenyl)-5'-morpholino-spiro [-bicyclo [2,2,1] heptan-2,2'-5-endo-pyrrolin]-D-hydrogentartrat

Das nach Beispiel 14 aus

Ethanol anfallende (±)-3-exo-(4-Chlorphenyl)-5'-morpholino-spiro [bicyclo [2,2,1] heptan-2,2',-5-endo-pyrrolin]-hydrojodid wird mit 2N Kalilauge/Methylenchlorid in die freie Base überführt.

12,3 g freie Base (hellgelbes Öl) werden in 400 ml Methanol gelöst und unter Rühren langsam mit 5,8 g D(−)-Weinsäure in 200 ml Wasser versetzt. Nach 2 h wird der feine Niederschlag abgesaugt. Der Feststoff wird aus 500 ml wässrigem Methanol (1 : 1) umkristallisiert. Farblose Blättchen, die sich bei 270 °C zersetzen. $[a]^{D}_{22}$ = − 106° (Methanol/$H_2O$ = 3 : 1).

Drehwert für freie Base : $[a]^{D}_{22}$ = − 126° (Methanol/$H_2O$ = 3 : 1). Auf ähnliche Weise wurde unter Modifizierung des vorstehenden Auflösungsverfahrens erhalten :

(+)-3-exo-(4-Chlorphenyl)-5'-morpholino-spiro-[bicyclo [2,2,1] heptan-2,2'-5-endo-pyrrolin]-L-hydrogentartrat,

farblose Blättchen vom Zersetzungspunkt 270 °C, $[a]^{D}_{22}$ = + 106° (Methanol/$H_2O$ = 3 : 1).

Beispiel 49

(−)-3-exo-(4-Fluorphenyl)-5'-morpholino-spiro-[bicyclo [2,2,1] heptan-2,2'-5-endo-pyrrolin]-D-hydrogentartrat

Das nach Beispiel 22 aus

Ethanol anfallende D,L-3-exo-(4-Fluorphenyl)-5'-morpholinospiro [bicyclo [2.2.1] heptan-2,2'-5-endo-pyrrolin] hydrojodid wird durch Ausschütteln mit 2 N Kalilauge/Methylenchlorid wie üblich in die salzfreie Form gebracht. Das nach Trocknen, Abfiltrieren und Eindampfen der organ. Phase anfallende Öl (10,7 g) wird in 185 ml Methanol gelöst und 4,9 g D (−)-Weinsäure in 60 ml Wasser langsam unter Rühren zugegeben. Nach 2 Std. Nachrühren wird abgesaugt und der Feststoff aus wässrigem Methanol (1 : 1) umkristallisiert. Farblose Kristalle vom Schmp. 256 °C (Zers.), $[a]_D^{22}$ = − 76° (Methanol/Wasser = 3 : 1).

In analoger Weise wird unter Modifizierung der vorstehenden Racematspaltung das (+)-3-exo-(4-Fluorphenyl)-5'-morpholino-spiro [bicyclo [2.2.1]-heptan-2,2'-5-endo-pyrrolin]-L-hydrogentartrat erhalten. Farblose Blättchen vom Zersetzungspunkt 255 °C, $[a]_D^{22}$ = − 69° (Methanol/Wasser = 3 : 1).

Beispiel 50

(−)-3-exo-(3,4-Dichlorphenyl)-5'-morpholino-spiro [bicyclo [2,2,1]-heptan-2,2'-5-endo-pyrrolin]-D-hydrogentartrat

Das nach Beispiel 24 aus

Ethanol anfallende (±)-exo-(3,4-Dichlorphenyl)-5'-morpholinospiro [bicyclo-[2.2.1] heptan-2,2'-5-endo-pyrrolin] hydrojodid wird analog zu Beispiel 48 zunächst in die salzfreie Form überführt und einer Racematspaltung mit D-Weinsäure unterworfen. Farblose Kristalle vom Schmp. 236 °C (Zers.) $[a]_D^{22}$ = − 101° (Methanol/Wasser = 3 : 1).

**Ansprüche** (für die Vertragsstaaten : BE, CH, DE, FR, GB, IT, NL, SE)

1. Heterocyclische, spiroverknüpfte Amidine der Formel I

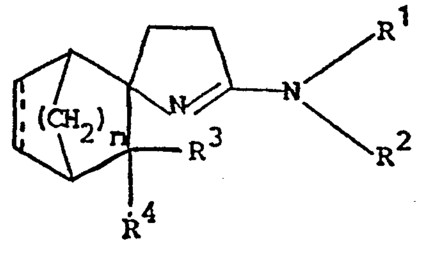

(I)

worin

a) n = 1 oder 2 ist ;

b) $R^1$ und $R^2$ Alkylreste sind, die gemeinsam mit dem Stickstoffatom einen 5-, 6- oder 7-gliedrigen Ring bilden, worin eines der Kohlenstoffatome auch durch ein Sauerstoff-, Schwefel- oder Stickstoffatom ersetzt sein kann, wobei das Stickstoffatom substituiert sein kann durch Wasserstoff oder einen Phenylrest, der seinerseits ein- oder mehrfach durch eine $C_1$-$C_4$-Alkyl- oder Alkyloxy-, Methylendioxy-, Hydroxy-, Halogen-, Nitro- oder Aminogruppe substituiert sein kann

c) $R^3$ = Wasserstoff oder Methyl ist und

d) $R^4$ einen gegebenenfalls durch $C_1$-$C_4$-Alkyl-, $C_1$-$C_4$-Alkoxy-, Halogen, oder Trifluormethyl-, ein- oder zweifach substituierten Phenylrest bedeutet,

sowie die physiologisch verträglichen Salze von Verbindungen der Formel I.

2. Stereoisomere exo-Aryl-endo-pyrrolin-Amidine nach Anspruch 1 der allgemeinen Formel XII

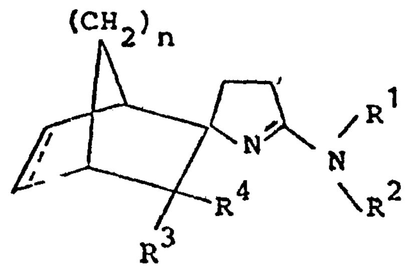

(XII)

worin $R^1$, $R^2$, $R^3$, $R^4$ und n die zu Anspruch 1 genannte Bedeutung haben, sowie ihre physiologisch verträglichen Salze.

3. Stereoisomere endo-Aryl-exo-pyrrolin-Amidine nach Anspruch 1 der allgemeinen Formel XVII

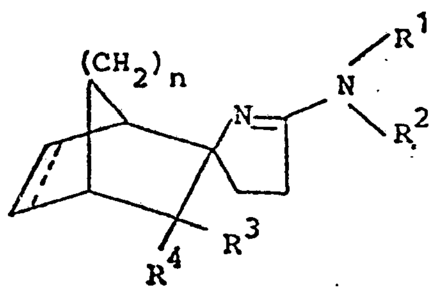

(XVII)

worin $R^1$, $R^2$, $R^3$, $R^4$ und n die zu Anspruch 1 genannte Bedeutung haben, sowie ihre physiologisch verträglichen Salze.

4. Optische Isomere der Formel XII und XVII gemäß Anspruch 2 und 3, sowie ihre physiologisch verträglichen Salze.

5. Linksdrehende Enantiomere der optischen Isomeren gemäß Anspruch 4, sowie ihre physiologisch verträglichen Salze.

6. Verfahren zur Herstellung von Verbindungen gemäß Anspruch 1 bis 5, dadurch gekennzeichnet, daß man

a) eine Verbindung der Formel III

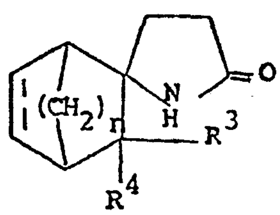

(III)

worin n, $R^3$ und $R^4$ die zur Formel I genannten Bedeutungen haben, mit Phosphoroxichlorid bzw. Phosphorpentoxid und einem Amin der Formel IV

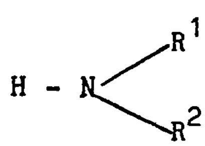

(IV)

worin $R^1$ und $R^2$ die zur Formel I genannte Bedeutung haben, umsetzt ; oder

b) eine Verbindung der Formel II

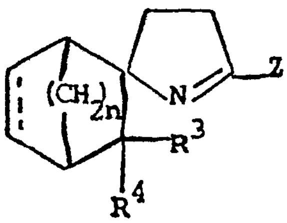

(II)

worin n, $R^3$ und $R^4$ die zur Formel I genannten Bedeutungen haben und Z ein Halogenatom oder ein SH, $SR^5$ oder $OR^5$ bedeutet, wobei $R^5$ einen $C_1$-$C_6$-Alkyl-, einen Benzyl-, Phenylalkyl- oder Phenylsulfonylrest darstellt mit einem Amin der Formel IV umsetzt ; oder

c) eine Verbindung der Formel V

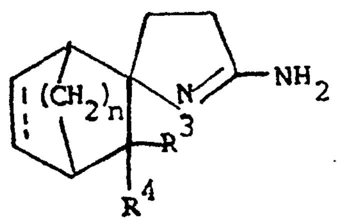

(V)

mit einem Alkylierungsmittel umsetzt ; oder

d) eine Verbindung der Formel III in trockenem Pyridin bei einer Temperatur von − 30 °C bis Raumtemperatur, vorzugsweise bei − 10 °C mit einem Arylsulfochlorid zu dem intermediären Iminoester umsetzt, der mit der Aminokomponente der Formel IV weiterreagiert.

7. Verfahren zur Herstellung der Verbindungen der Formel XVII nach Anspruch 3, dadurch gekennzeichnet, daß zur Synthese nach Anspruch 6 die aus der Mutterlauge des exo-Aryl-endo-nitro-Esters der Formel XV

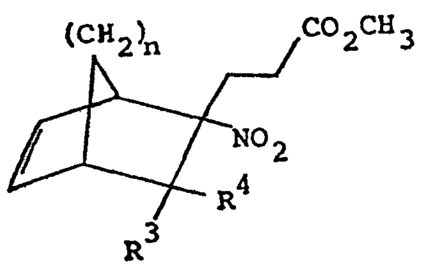

(XV)

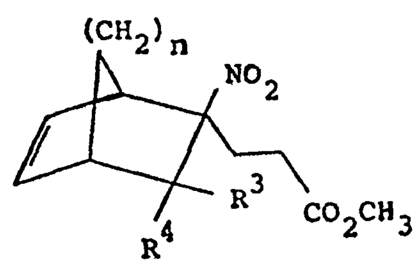

(XVI)

säulenchromatographisch isolierte Verbindung der Formel XVI eingesetzt wird, in der n, $R^3$ und $R^4$ die in Anspruch 1 genannten Bedeutungen haben.

8. Verfahren zur Herstellung der optischen Isomeren gemäß Ansprüchen 4 und 5, dadurch gekennzeichnet, daß man ein Razemat der in den Ansprüchen 2 und 3, angegebenen Formeln XII oder XVII oder eine racemische Vorstufe der Formeln II, III oder V nach einem Standardverfahren in die optischen Isomeren aufspaltet und erforderlichenfalls eine so erhaltene optisch aktive Verbindung in eine der Verbindungen der Formeln XII oder XVII umwandelt.

9. Verfahren nach Anspruch 8, dadurch gekennzeichnet, daß das linksdrehende Isomere abgetrennt wird.

10. Pharmazeutische Zubereitung, dadurch gekennzeichnet, daß sie eine Verbindung der Ansprüche 1-5 und einen pharmazeutisch verträglichen Träger enthält.

11. Pharmazeutische Zubereitung nach Anspruch 10, insbesondere zur oralen oder parenteralen Verabreichung, dadurch gekennzeichnet daß sie als Wirkstoff mindestens ein optisches Isomeres gemäß Anspruch 4 oder 5 in Verbindung mit pharmazeutisch verträglichen Hilfs- und Trägerstoffen enthält.

**Ansprüche** (für den Vertragsstaat AT)

1. Verfahren zur Herstellung von heterocyclischen spiroverknüpften Amidinen der Formel I

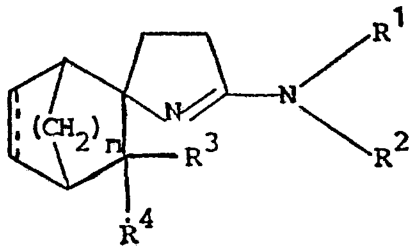

(I)

und ihren physiologisch verträglichen Salzen
worin

a) n = 1 oder 2 ist ;

b) $R^1$ und $R^2$ Alkylreste sind, die gemeinsam mit dem Stickstoffatom einen 5-, 6- oder 7-gliedrigen Ring bilden, worin eines der Kohlenstoffatome auch durch ein Sauerstoff-, Schwefel- oder Stickstoffatom ersetzt sein kann, wobei das Stickstoffatom substituiert sein kann durch Wasserstoff oder einen Phenylrest, der seinerseits ein- oder mehrfach durch eine $C_1$-$C_4$-Alkyl- oder Alkyloxy-, Methylendioxy-, Hydroxy-, Halogen-, Nitro- oder Aminogruppe substituiert sein kann

c) $R^3$ = Wasserstoff oder Methyl ist und

d) $R^4$ einen gegebenenfalls durch $C_1$-$C_4$-Alkyl-, $C_1$-$C_4$-Alkoxy-, Halogen-, oder Trifluormethyl-, ein- oder zweifach substituierten Phenylrest bedeutet, dadurch gekennzeichnet, daß man

a) eine Verbindung der Formel III

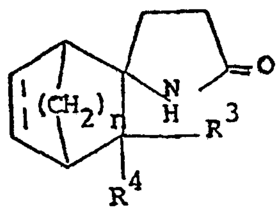

(III)

worin n, $R^3$ und $R^4$ die zur Formel I gennanten Bedeutungen haben, mit Phosphoroxichlorid bzw. Phosphorpentoxid und einem Amin der Formel IV

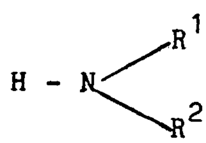

(IV)

worin $R^1$ und $R^2$ die zur Formel I genannte Bedeutung haben, umsetzt ; oder

b) eine Verbindung der Formel II

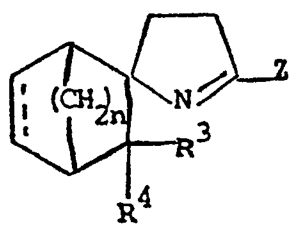

(II)

worin n, $R^3$ und $R^4$ die zur Formel I genannten Bedeutungen haben und Z ein Halogenatom oder ein SH, $SR^5$ oder $OR^5$ bedeutet, wobei $R^5$ einen $C_1$-$C_6$-Alkyl-, einen Benzyl-, Phenylalkyl- oder Phenylsulfonylrest darstellt, mit einem Amin der Formel IV umsetzt ; oder

c) eine Verbindung der Formel V

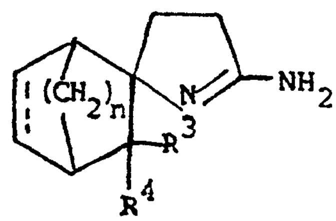

(V)

mit einem Alkylierungsmittel umsetzt ; oder

d) eine Verbindung der Formel III in trockenem Pyridin bei einer Temperatur von – 30 °C bis Raumtemperatur, vorzugsweise bei – 10 °C mit einem Arylsulfochlorid zu dem intermediären Iminoester umsetzt, der mit der Aminokomponente der Formel IV weiterreagiert.

2. Verfahren zur Herstellung von endo-Aryl-endo-pyrrolin-Amidinen der Formel XVII

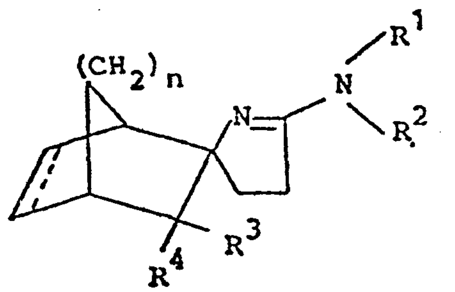

(XVII)

und ihrer physiologisch verträglichen Salze, dadurch gekennzeichnet, daß zur Synthese nach Anspruch 1 die aus der Mutterlauge des exo-Aryl-endo-nitroesters der Formel XV

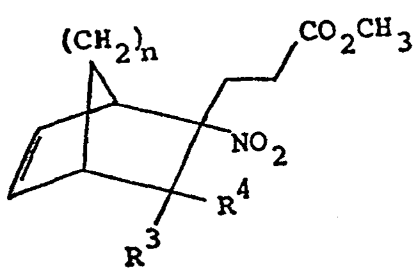

(XV)

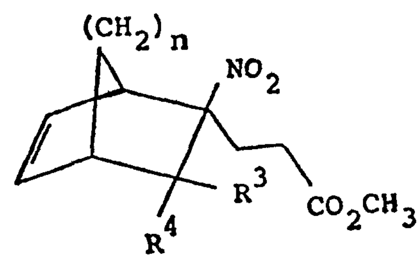

(XVI)

säulenchromatographisch isolierte Verbindung der Formel XVI eingestetzt wird, in der n, $R^3$ und $R^4$ die in Anspruch 1 genannten Bedeutungen haben.

3. Verfahren zur Herstellung von optischen Isomeren der nach Anspruch 1 gewonnenen Verbindungen oder ihrer physiologisch verträglichen Salze, dadurch gekennzeichnet, daß man ein Razemat dieser Verbindungen der Formeln XII oder XVII

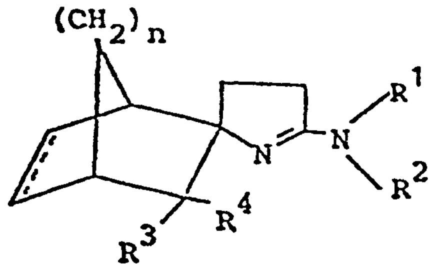

(XII)

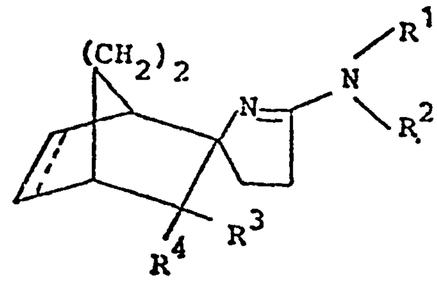

(XVII)

oder eine ihrer racemischen Vorstufe der Formeln II, III oder V nach einem bekannten Verfahren in die optischen Isomeren der Verbindungen XII oder XVII aufspaltet oder die so gewonnene optisch aktive Verbindung nach dem Verfahren von Anspruch 1 in die Verbindungen XII oder XVII umwandelt.

4. Verfahren nach Anspruch 3, dadurch gekennzeichnet, daß das linksdrehende Isomere abgetrennt wird.

5. Verfahren zur Herstellung einer pharmazeutischen Zubereitung, dadurch gekennzeichnet, daß man ein Stereoisomeres der Formeln XII oder XVII mit einem pharmazeutisch verträglichen Träger zu einem Arzneimittel verarbeitet.

6. Verfahren zur Herstellung einer pharmazeutischen Zubereitung, insbesondere zur oralen oder parenteralen Verabreichung, dadurch gekennzeichnet, daß man mindestens ein optisches Isomeres, das gemäß Anspruch 4 hergestellt wurde, mit pharmazeutisch verträglichen Hilfs- und Trägerstoffen zu einem Arzneimittel verarbeitet.

**Claims** (for the contracting States : BE, CH, DE, FR, GB, IT, NL, SE)

1. Heterocyclic, spiro-linked amidines of the formula I

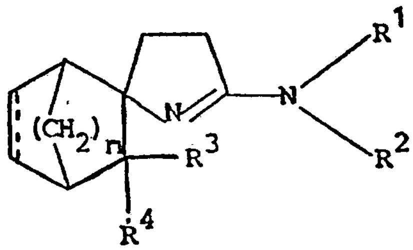

(I)

in which

a) n is 1 or 2 ;

b) $R^1$ and $R^2$ are hydrogen or alkyl radicals which, together with the nitrogen atom, can form a 5-, 6- or 7-membered ring, in which one of the carbon atoms can also be replaced by an oxygen, sulfur on nitrogen atoms, it being possible for the nitrogen atom to be substituted by hydrogen or a phenyl radical, which can in turn be monosubstituted or polysubstituted by a $C_1$ to $C_4$-alkyl group or an alkoxy, methylenedioxy, hydroxy, halogen, nitro or amino group,

c) $R^3$ is hydrogen or methyl and

d) $R^4$ denotes a phenyl radical which is optionally monosubstituted or disubstituted by $C_1$-$C_4$-alkyl, $C_1$-$C_4$-alkoxy, halogen or trifluoromethyl, and the physiologically acceptable salts of compounds of the formula I.

2. Stereoisomeric exo-aryl-endo-pyrroline amidines as claimed in claim 1, of the general formula XII

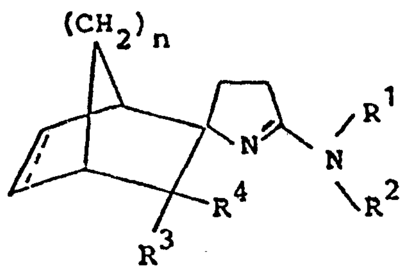

(XII)

in which $R^1$, $R^2$, $R^3$, $R^4$ and n have the meaning given for claim 1, and their physiologically acceptable salts.

3. Stereoisomeric endo-aryl-exo-pyrroline amidines as claimed in claim 1, of the general formula XVII

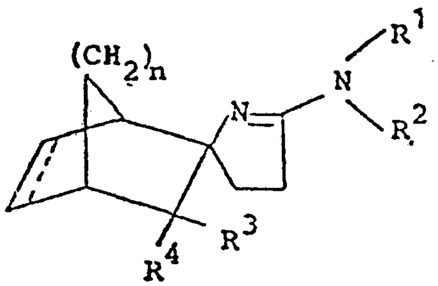

(XVII)

in which $R^1$, $R^2$, $R^3$, $R^4$ and n have the meaning given for claim 1, and their physiologically acceptable salts.

4. Optical isomers of the formula XII and XVII as claimed in claims 2 and 3, and their physiologically acceptable salts.

5. Laevo-rotatory enantiomers of the optical isomers as claimed in claim 4, and their physiologically acceptable salts.

6. Process for the preparatiion of compounds as claimed in claims 1 to 5, which comprises

a) reacting a compund of the formula III

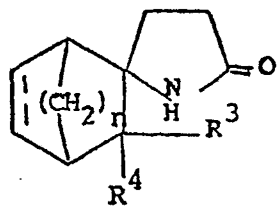

(III)

in which n, $R^3$ and $R^4$ have the meanings given for formula I, with phosphorus oxychloride or phosphorus pentoxide and an amine of the formula IV

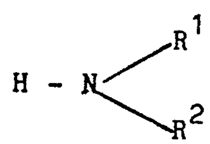

(IV)

in which $R^1$ and $R^2$ have the meaning given for formula I ; or

b) reacting a compound of the formula II

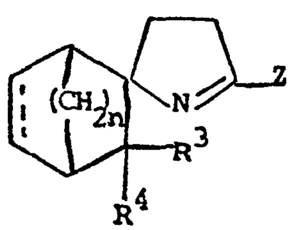

(II)

in which n, $R^3$ and $R^4$ have the meanings given for formula I and Z denotes a halogen atom or a SH, $SR^5$ or $OR^5$ radical, in which $R^5$ represents a $C_1$-$C_6$-alkyl radical or a benzyl, phenylalkyl or phenylsulfonyl radical, with an amine of the formula IV ; or

c) reacting a compound of the formula V

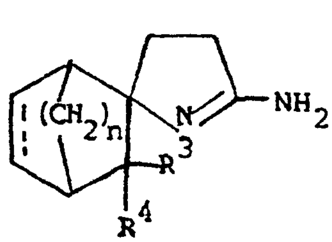

(V)

with an alkylating agent ; or

d) reacting a compound of the formula III with an arylsulfonyl chloride in dry pyridine at a temperature of – 30 °C to room temperature, preferably at – 10 °C, to give the intermediate imino-ester, which reacts further with the amine component of the formula IV.

7. Process for the preparation of the compounds of the formula XVII as claimed in claim 3, which comprises using for the synthesis as claimed in claim 6 the compound of the formula XVI, in which n, $R^3$ and $R^4$ have the meanings given in claim 1, isolated, by column chromatography, from the mother liquor of the exo-aryl-endo-nitro-ester of the formula XV

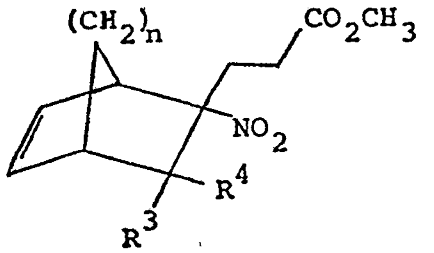

(XV)

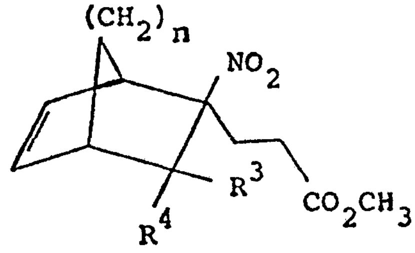

(XVI)

8. Process for the preparation of the optical isomers as claimed in claims 4 and 5, which comprises resolving a racemate of the formulae XII and XVII indicated in claims 2 and 3, or a racemic precursor thereof of the formulae II, III or V, into the optical isomers by a standard process and, if necessary, converting an optically active compound thus obtained into one of the compounds of the formula XII or XVII.

9. Process as claimed in claim 8, wherein the laevorotatory isomer is separed off.

10. Pharmaceutical formulation, which contains a compound of claims 1-5 and a pharmaceutically acceptable exipient.

11. Pharmaceutical formulation as claimed in claim 10, in particular for oral or parenteral administration, which contains, as the active compound, at least one optical isomer as claimed in claim 4 or 5, in association with pharmaceutically acceptable auxiliaries and excipients.

**Claims** (for the contracting State AT)

1. Process for the preparation of heterocyclic, spirolinked amidines of the formula I

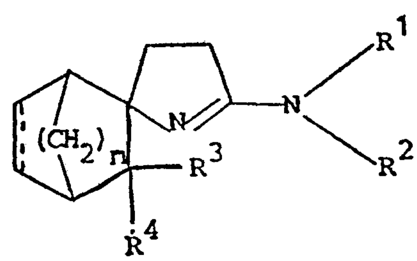

(I)

in which

a) n is 1 or 2;

b) $R^1$ and $R^2$ are alkyl radicals with, together with the nitrogen atom, can form a 5-, 6- or 7-membered ring, in which one of the carbon atoms can also be replaced by an oxygen, sulfur or nitrogen atom, it being possible for the nitrogen atom to be substituted by hydrogen or a phenyl-radical, which can in turn be monosubstituted or polysubstituted by a $C_1$ to $C_4$-alkyl group or an alkoxy, methylenedioxy, hydroxy, halogen, nitro or amino group,

c) $R^3$ is hydrogen or methyl and

d) $R^4$ denotes a phenyl radical which is optionally monosubstituted or disubstituted by $C_1$-$C_4$-alkyl, $C_1$-$C_4$-alkoxy, halogen or trifluoromethyl, and of their physiologically acceptable salts; which comprises

a) reacting a compound of the formula III

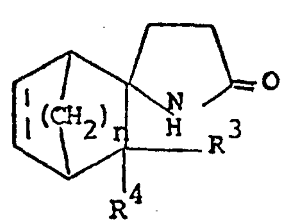

(III)

in which n, $R^3$ and $R^4$ have the meanings given for formula I, with phosphorus oxychloride or phosphorus pentoxide and an amine of the formula IV

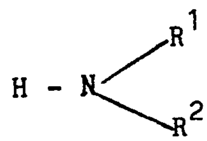

(IV)

in which $R^1$ and $R^2$ have the meaning given for formula I ; or

b) reacting a compound of the formula II

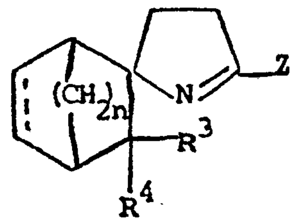

(II)

in which n, $R^3$ and $R^4$ have the meanings given for formula I and Z denotes a halogen atom or a SH, $SR^5$ or $OR^5$ radical, in which $R^5$ represents a $C_1$-$C_6$-alkyl radical or a benzyl, phenylalkyl or phenylsulfonyl radical, with an amine of the formula IV ; or

c) reacting a compound of the formula V

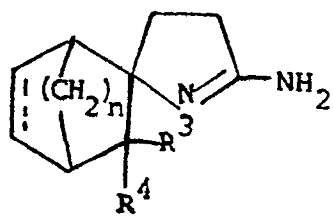

(V)

with an alkylating agent ; or

d) reacting a compound of the formula III with an aryl-sulfonyl chloride in dry pyridine at a temperature of – 30 °C to room temperature, preferably at – 10 °C, to give the intermediate imino-ester, which reacts further with the amine component of the formula IV.

2. Process for the preparation of endo-aryl-exo-pyrroline amidines of the general formula XVII

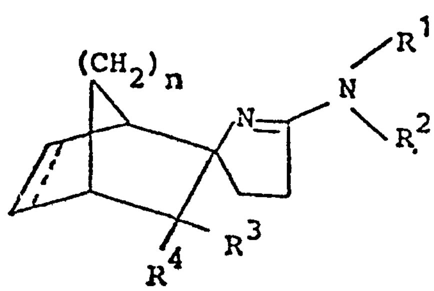

(XVII)

and their physiologically acceptable salts, which comprises using for the synthesis as claimed in claim 1, the compound of the formula XVI, in which n, $R^3$ and $R^4$ have the meanings given in claim 1, isolated, by column chromatography, from the mother liquor of the exo-aryl-endo-nitro-ester of the formula XV

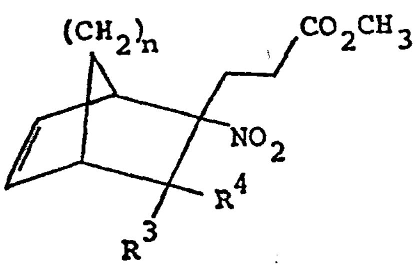

(XV)

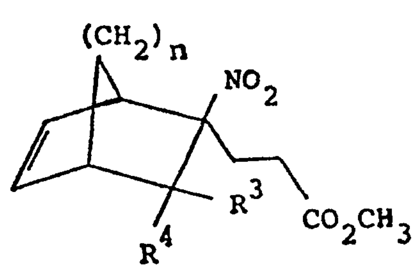

(XVI)

3. Process for the preparation of optical isomers of the compounds obtained according to claim 1, which comprises resolving a racemate of these compounds of the formulae XII and XVII

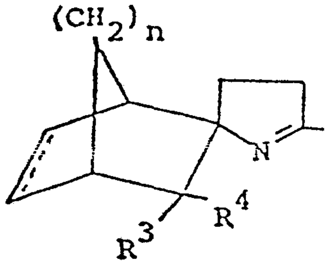

(XII)

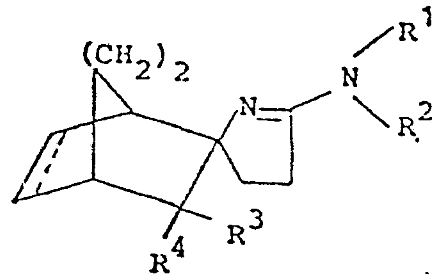

(XVII)

or a racemic precursor thereof of the formulae II, III or V, into the optical isomers of the compounds XII or XVII by a standard process and, if necessary, converting an optically active compound thus obtained into one of the compounds of the formula XII or XVII, according to the process of claim 1.

4. Process as claimed in claim 3, wherein the laevo-rotatory isomer is separated off.

5. Process for the preparation of a pharmaceutical formulation, which comprises processing a stereoisomer of the formula XII or XVII with a pharmaceutically acceptable excipient to yield a medicament.

6. Process for the preparation of a pharmaceutical formulation, in particular for oral or parenteral administration, which comprises processing at least one optical isomer, that has been prepared according to claim 4, with pharmaceutically acceptable auxiliaries and excipients to yield a medicament.

**Revendications** (pour les Etats contractants : BE, CH, DE, FR, GB, IT, NL, SE)

1. Spiro-amidines hétérocycliques de formule I

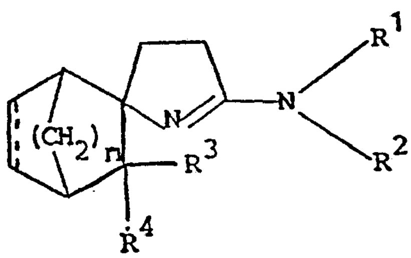

(I)

dans laquelle :

a) n = 1 ou 2 ;

b) $R^1$ et $R^2$ sont des radicaux alcoyle qui forment avec l'atome d'azote un cycle à 5, 6 ou 7 chaînons, dans lequel un des atomes de carbone peut également être remplacé par un atome d'oxygène, de soufre ou d'azote, l'atome d'azote pouvant être substitué par de l'hydrogène ou par un radical phényle qui peut de son côté être substitué une ou plusieurs fois par un groupe alcoyle en $C_1$ à $C_4$ ou un groupe alcoyloxy, méthylènedioxy, hydroxy, halogéno, nitro ou amino,

c) $R^3$ est l'hydrogène ou un groupe méthyle, et

d) $R^4$ est un radical phényle substitué le cas échéant une ou deux fois par un groupe alcoyle en $C_1$ à $C_4$, alcoxy en $C_1$ à $C_4$, halogéno ou trifluorométhyle, ainsi que les sels physiologiquement acceptables de composés de formule I.

2. Exo-aryl-endo-pyrroline-amidines stéréoisomères selon la revendication 1, de formule générale XII

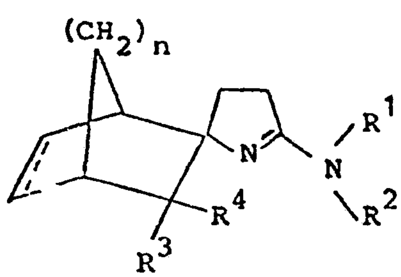

(XII)

dans laquelle $R^1$, $R^2$, $R^3$, $R^4$ et n ont les significations indiquées à la revendication 1, et leurs sels physiologiquement acceptables.

3. Endo-aryl-exo-pyrroline-amidines stéréoisomères suivant la revendication 1, de formule générale XVII

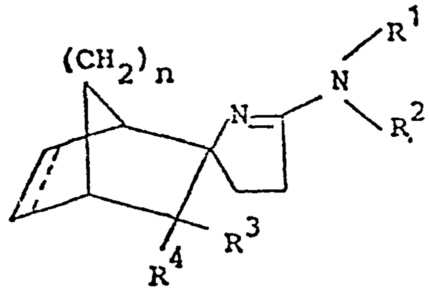

(XVII)

dans laquelle $R^1$, $R^2$, $R^3$, $R^4$ et n ont les significations indiquées à la revendication 1, et leurs sels physiologiquement acceptables.

4. Isomères optiques répondant aux formules XII et XVII suivant l'une quelconque des revendications 2 et 3, et leurs sels physiologiquement acceptables.

5. Enantiomères lévogyres des isomères optiques suivant la revendication 4, et leurs sels physiologiquement acceptables.

6. Procédé de préparation de composés suivant l'une quelconque des revendications 1 à 5, caractérisé en ce que :

a) on fait réagir un composé de formule III

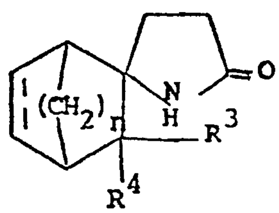

(III)

dans laquelle n, $R^3$ et $R^4$ ont les significations indiquées à propos de la formule I, avec de l'oxychlorure de phosphore ou du pentoxyde de phosphore et une amine de formule IV

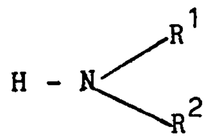

(IV)

dans laquelle $R^1$ et $R^2$ ont les significations indiquées à propos de la formule I ; ou

b) on fait réagir un composé de formule II

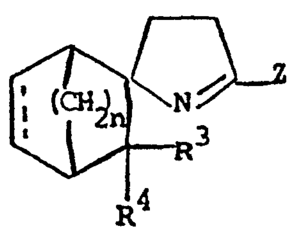

(II)

dans laquelle n, $R^3$ et $R^4$ ont les significations indiquées à propos de la formule I et Z désigne un atome d'halogène ou un groupe SH, $SR^5$ ou $OR^5$, ou $R^5$ représente un radical alcoyle en $C_1$ à $C_6$, un radical benzyle, phénylalcoyle ou phénylsulfonyle, avec une amine de formule IV ; ou

c) on fait réagir un composé de formule V

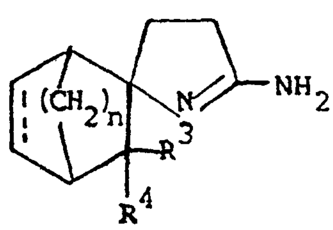

(V)

avec un agent d'alcoylation ; ou

d) on fait réagir un composé de formule III dans de la pyridine sèche à une température comprise entre – 30 °C et la température ambiante, de préférence à – 10 °C, avec un sulfochlorure d'aryle pour donner l'iminoester, qui réagit ultérieurement avec le composé aminé de formule IV.

7. Procédé de préparation de composés de formule XVII suivant la revendication 3, caractérisé en ce qu'on utilise, pour la synthèse suivant la revendication 6, le composé de formule XVI isolé par chromatographie sur colonne de la liqueur-mère de l'exo-aryl-nitro-ester de formule XV

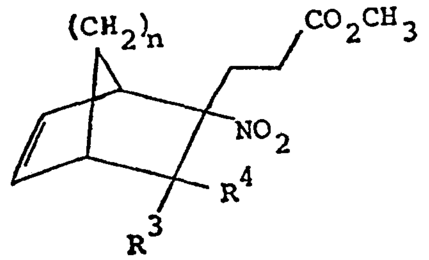

(XV)

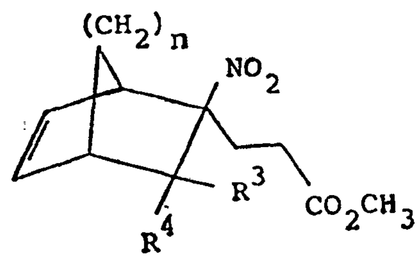

(XVI)

n, $R^3$ et $R^4$ ayant les significations indiquées dans la revendication 1.

8. Procédé de préparation des isomères optiques suivant l'une quelconque des revendications 4 et 5, caractérisé en ce qu'on dédouble un racémate répondant aux formules XII ou XVII indiquées dans les revendications 2 et 3, ou un stade antérieur racémique répondant aux formules II, III ou V, par un procédé ordinaire, en ses isomères optiques et en ce qu'on transforme le cas échéant un composé optiquement actif ainsi obtenu en un des composés répondant aux formules XII ou XVII.

9. Procédé suivant la revendication 8, caractérisé en ce qu'on sépare l'isomère lévogyre.

10. Préparation pharmaceutique, caractérisée en ce qu'elle contient un composé suivant l'une quelconque des revendications 1 à 5 et un véhicule pharmaceutiquement acceptable.

11. Préparation pharmaceutique suivant la revendication 10, en particulier pour l'administration orale ou parentérale, caractérisée en ce qu'elle contient comme substance active au moins un isomère optique suivant l'une quelconque des revendications 4 et 5, associé à des adjuvants et à des véhicules pharmaceutiquement acceptables.

**Revendications** (pour l'Etat contractant AT)

1. Procédé de préparation de spiro-amidines hétérocycliques de formule I

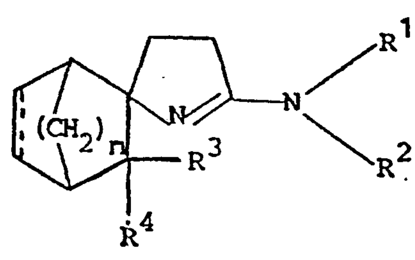

(I)

dans laquelle :

a) n = 1 ou 2 ;

b) $R^1$ et $R^2$ sont des radicaux alcoyle qui forment avec l'atome d'azote un cycle à 5, 6 ou 7 chaînons, dans lequel un des atomes de carbone peut également être remplacé par un atome d'oxygène, de soufre ou d'azote, l'atome d'azote pouvant être substitué par de l'hydrogène ou par un radical phényle qui peut de son côté être substitué une ou plusieurs fois par un groupe alcoyle en $C_1$ à $C_4$ ou un groupe alcoyloxy, méthylènedioxy, hydroxy, halogéno, nitro ou amino,

c) $R^3$ est l'hydrogène ou un groupe méthyle, et

d) $R^4$ est un radical phényle substitué le cas échéant une ou deux fois par un groupe alcoyle en $C_1$ à $C_4$, alcoxy en $C_1$ à $C_4$, halogéno ou trifluorométhyle, et de leurs sels physiologiquement acceptables, procédé caractérisé en ce que :

a) on fait réagir un composé de formule III :

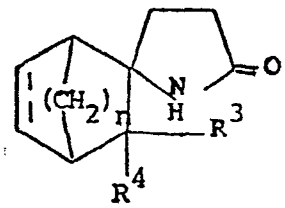

(III)

dans laquelle n, $R^3$ et $R^4$ ont les significations indiquées à propos de la formule I, avec de l'oxychlorure de phosphore ou du pentoxyde de phosphore et une amine de formule IV

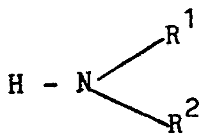

(IV)

dans laquelle $R^1$ et $R^2$ ont les significations indiquées à propos de la formule I ; ou

b) on fait réagir un composé de formule II

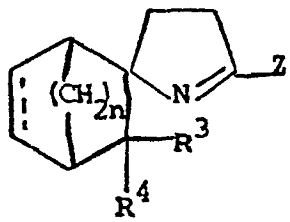

(II)

dans laquelle n, $R^3$ et $R^4$ ont les significations indiquées à propos de la formule I et Z désigne un atome d'halogène ou un groupe SH, $SR^5$ ou $OR^5$, ou $R^5$ représente un radical alcoyle en $C_1$ à $C_6$, un radical benzyle, phénylalcoyle ou phénylsulfonyle, avec une amine de formule IV ; ou

c) on fait réagir un composé de formule V

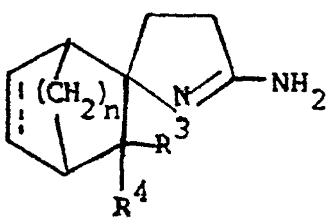

(V)

avec un agent d'alcoylation ; ou

d) on fait réagir un composé de formule III dans de la pyridine sèche à une température comprise entre − 30 °C et la température ambiante, de préférence à − 10 °C, avec un sulfochlorure d'aryle pour donner l'iminoester, qui réagit ultérieurement avec le composé aminé de formule IV.

2. Procédé de préparation d'endo-aryl-exo-pyrroline-amidines de formule générale XVII

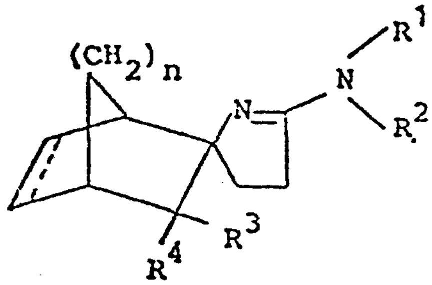

(XVII)

et de leurs sels physiologiquement acceptables, caractérisé en ce qu'on utilise, pour la synthèse suivant la revendication 1, le composé de formule XVI isolé par chromatographie sur colonne de la liqueur-mère de l'exo-aryl-nitro-ester de formule XV

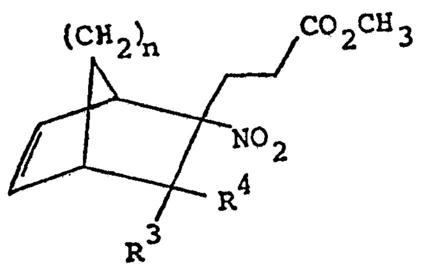
(XV)

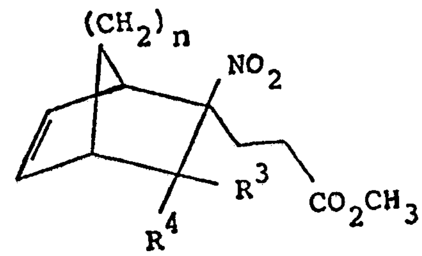
(XVI)

n, $R^3$ et $R^4$ ayant les significations indiquées dans la revendication 1.

3. Procédé de préparation d'isomères optiques des composés obtenus suivant la revendication 1 ou de leurs sels physiologiquement acceptables, caractérisé en ce qu'on dédouble un racémate de ces composés répondant aux formules XII ou XVII

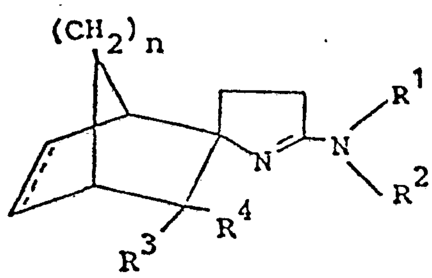
(XII)

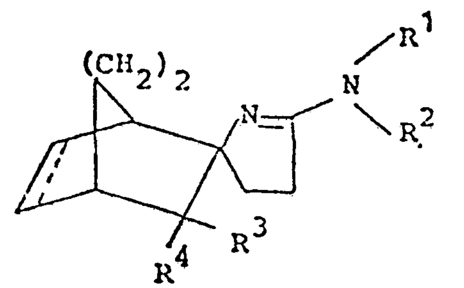
(XVII)

ou un stade antérieur racémique répondant aux formules II, III ou V, par un procédé ordinaire, en les isomères optiques des composés répondant aux formules XII ou XVII et en ce qu'on transforme le cas échéant un composé optiquement actif ainsi obtenu en un des composés optiquement actifs ainsi obtenu en un des composés répondant aux formules XII ou XVII, par le procédé suivant la revendication 1.

4. Procédé suivant la revendication 3, caractérisé en ce qu'on sépare l'isomère lévogyre.

5. Procédé de préparation d'un produit pharmaceutique, caractérisé en ce qu'on traite un stéréoisomère répondant aux formules XII ou XVII par un véhicule pharmaceutiquement acceptable pour obtenir un médicament.

6. Procédé de préparation d'un produit pharmaceutique, en particulier pour l'administration orale ou parentérale, caractérisé en ce qu'on traite au moins un isomère optique obtenu selon la revendication 4 par des adjuvants et véhicules pharmaceutiquement acceptables pour obtenir un médicament.